# EUROPEAN PATENT APPLICATION

(11) **EP 2 308 377 A1**
(43) Date of publication of application: **13.04.2011**
(21) Application number: 08752396.5
(22) Date of filing: 07.05.2008
(51) Int. Cl.: A61B 10/00, G01N 27/30

(54) **DEVICE FOR DIAGNOSING TISSUE INJURY**

(71) Applicant: Tokyo University of Science, Tokyo 162-8601 (JP); Yamaguchi University, Yamaguchi 753-8511 (JP); Acteiive Corporation, Chiba 278-8510 (JP)
(72) Inventor: YUASA, Makoto, Tokyo 162-8601 (JP); MAEKAWA, Tsuyoshi, Ube-shi Yamaguchi 755-8505 (JP); FUJITA, Motoki, Ube-shi Yamaguchi 755-8505 (JP); KIDO, Shigeru, Ishikawa-gun Fukushima 963-7808 (JP)
(74) Representative: McIlroy, Steven David
(86) International application number: PCT/JP2008/058504
(87) International publication number: WO 2009/136436

(57) **Abstract**

The device for diagnosing tissue injury of the invention has a catheter insertable into the body and a radical sensor provided in the catheter, and is **characterized in that** the radical sensor has a sensor electrode capable of measuring superoxide anion radicals provided at a tip end of the catheter, a lead wire connector for a sensor provided at a basal portion of the catheter, and a lead wire for a sensor for connecting the sensor electrode portion to the lead wire connector for a sensor. With the device, in vivo free radicals typified by superoxide anions in systemic organ or tissue injury caused by cerebral ischemia reperfusion injury, severe infection or sepsis can be promptly and quantitatively monitored, and whether the in vivo tissue conditions are good or not can be accurately diagnosed.

## Description

### TECHNICAL FIELD

The present invention relates to a device for diagnosing tissue injury which diagnoses whether the in vivo tissue conditions are good or not, and particularly relates to a device for diagnosing tissue injury preferable for diagnosing whether the tissue conditions of a human body are good or not, and for diagnosing, for example, cerebral ischemia reperfusion injury, and systemic organ or tissue injury caused by severe infection or sepsis.

### BACKGROUND ART

Cerebral ischemia reperfusion injury in stroke is a brain disorder caused by reactive oxygen species, reactive nitrogen species, free radicals or the like in the body (hereinafter collectively referred to as "in vivo free radicals"). In addition, severe infection, sepsis or the like is caused by in vivo free radicals.

Among these in vivo free radicals, superoxide anion radical (O₂⁻•), hydrogen peroxide, hydroxyl radical, singlet oxygen, lipid peroxide radical and the like are known as reactive oxygen species (hereinafter referred to as "ROS"). Though ROS play various important roles in biophylaxis and homeostasis, production of excess ROS induced by oxidative stress induce radical toxicity, which leads to lipid peroxidation and apotosis. In addition, excess ROS are also produced in occlusion and reperfusion of organ blood vessels in stroke or the like, or in severe infection or sepsis.

Accordingly, there is a need for measurement of in vivo ROS in the field of clinical medicine. However, it is considered very difficult to make quantitative analysis of ROS, because ROS are quickly eliminated by antioxidase and antioxidant in the living body.

Meanwhile, it is known that most of ROS in the living body derive from superoxide anion radicals. Superoxide anion radical becomes extinct naturally by dismutation [2O₂⁻• + 2H⁺ -> O₂ + H₂O₂]. Metal porphyrin complex polymerized coating which has the same structure as the 6th coordination position of the heme in the active center of cytochrome c can catalyze the dismutation reaction of superoxide anion radicals, and the oxidation current can be detected.

The linear correlation between the amount of superoxide anion radicals and the detection current has been confirmed by the experiment of xanthine - xanthine oxidase reaction which generates superoxide anion radicals. In addition, it has also been confirmed that the detection current of superoxide anion radicals decreased promptly by administration of superoxide dismutase (SOD) and that the electrode detects only superoxide anion radicals selectively and does not detect H₂O₂. This is important for making quantitative analysis of superoxide anion radicals on real time in the living body.

A method, such as superoxide spectroscopic measurement, which has been conventionally conducted as a method for measuring anion radicals in the living body, cannot be used on real time, and thus use of it practically is not preferable, or difficult. Although biosensors using the electrode catalyst using organic substance such as cytochrome c, SOD, etc. as an in vivo measurement method or as a method aimed at in vivo measurement (see Non-patent Literatures 1 to 4 to be described later), there is also drawbacks for long-term stability and their repetitive use. Modified electrode by absorbing the active center of cytochrome c as a non-enzymatic sensor has a problem reaction selectivity.

The inventor Yuasa et al. already developed an electrochemical sensor using metal porphyrin complex polymerized coating as a cytochrome c mimic. This sensor had fine selectivity of superoxide anion radicals, and was able to make quantitative analysis of ROS on real time (see Patent literature 1 and Non-patent Literature 5 to be described later). Furthermore, the inventor has also proposed a method for enhancing biocompatibility using this sensor (see Patent Literature 2 to be described later).

Patent Literature 1: PCT Patent Application No. WO03/054536
Patent Literature 2: Japanese Patent Laid-open Publication No. 2006-314386
Non-patent Literature 1: Cooper JM, Greenough KR, McNeil CJ: "J. Electroanal Chem.", 347, 267-275 (1993)
Non-patent Literature 2: Tian Y, Mao L, Okajima T, et al.,: "Amal. Chem.", 74 (10), 2428-2434 (2002)
Non-patent Literature 3: Gobi KV, Mizutani F: "J. Electroanal Chem.", 484, 172-181 (2000)
Non-patent Literature 4: Beissenhirtz MK, Scheller FW, Lisdat F: "Amal. Chem.", 74 (10), 2428-2434 (2002)
Non-patent Literature 5: Yuasa M, Oyaizu K, Yammaguchi A, et al.,: "Polymers for Advanced Technologies", 16 (4), 287-292 (2005)

### DISCLOSURE OF INVENTION

### PROBLEMS TO BE RESOLVED BY THE INVENTION

If the above-described superoxide anion radical sensor proposed by the inventor, Yuasa et al., becomes available clinically, diseases and pathological conditions related to superoxide anion radicals will be able to be monitored, and the sensor will be extremely useful for their diagnosis and treatment. This is because there is a strong clinical need for development of superoxide anion radical sensor suitable for diseases and pathological conditions such as cerebral ischemia reperfusion injury. This is also because there is a need for accurate measurement of concentration of superoxide anion radicals and utilization of them in diagnosis also in systemic organ or tissue injury caused by severe infection or sepsis.

An object of the present invention is to provide a device for diagnosing tissue injury that is suitable for these purposes.

### MEANS OF SOLVING THE PROBLEMS

Therefore, through their extensive research, the inventor et al. have improved the electrode for the above-described superoxide anion radical sensor so as to be suitable for in vivo treatment, and enhanced biocompatibility by making the surface of the electrode harder so as to stand the use in the body chemically and mechanically. Furthermore, the inventor et al. used the superoxide anion radical sensor to evaluate property of superoxide anion radicals in jugular venous in forebrain ischemia rats and conducted microdialysis to evaluate cerebral damage. As a result, the inventor et al. have found that the obtained superoxide anion radical sensor is useful as a device for diagnosing tissue injury which diagnoses whether the in vivo tissue conditions are good or not. Particularly, they have found that the sensor is also useful as a device for cerebral ischemia reperfusion injury, wherein the tissue injury is cerebral ischemia reperfusion injury and further is cerebral ischemia reperfusion injury exacerbated by hyperglycemia (or diabetes or other disease caused by hyperglycemia). Furthermore, the inventor et al. have found that the sensor is useful as a device for diagnosing drug therapy, low-temperature therapy, high-concentration oxygen therapy or the like against cerebral ischemia reperfusion injury. Furthermore, as a result of the test using an acute phase sepsis model in the same manner, they have found that the above-described superoxide anion radical sensor is also useful in systemic organ or tissue injury caused by severe infection or sepsis, and finalized the present invention.

That is, the present invention has a catheter insertable into the body and a radical sensor provided in the catheter, and is **characterized in that** said radical sensor has a sensor electrode capable of measuring superoxide anion radicals provided at a tip end of said catheter, a lead wire connector for a sensor provided at a basal portion of said catheter, and a lead wire for a sensor for connecting said sensor electrode to the lead wire connector for a sensor.

Furthermore, the present invention is characterized by having comparative means for comparing a concentration of superoxide anion radicals in blood measured by said sensor electrode with a predetermined threshold to distinguish a tissue injury-based value and a healthy man-based value.

Furthermore, the present invention is **characterized in that** the sensor electrode capable of measuring superoxide anion radicals uses metal porphyrin complex polymerized coating.

Furthermore, the present invention is **characterized in that** transfusion supply means is provided in said catheter, and said transfusion supply means has tip end members provided at a tip end of said catheter and having a hole capable of discharging transfusion, transfusion lines each of which is connected to each of these tip end members, and transfusion connectors each of which is coupled to each of these transfusion lines and provided at a basal portion of said catheter.

Furthermore, the present invention is **characterized in that** at least one of said transfusion lines is coupled to a pressure transducer for venous pressure measurement.

Furthermore, the present invention is **characterized in that** a hole capable of discharging transfusion is also provided at a middle portion of the catheter.

Furthermore, the present invention is **characterized in that** the tissue injury is cerebral ischemia reperfusion injury.

Furthermore, the present invention is **characterized in that** the tissue injury is cerebral ischemia reperfusion injury, and further is cerebral ischemia reperfusion injury exacerbated by hyperglycemia (or diabetes or other disease caused by hyperglycemia).

Furthermore, the present invention is **characterized in that** the tissue injury is cerebral ischemia reperfusion injury and its treatment is drug therapy or low-temperature therapy.

Furthermore, the present invention is **characterized in that** the tissue injury is cerebral ischemia reperfusion injury and its treatment is high-concentration oxygen therapy.

Furthermore, the present invention is **characterized in that** the tissue injury is systemic organ or tissue injury caused by severe infection or sepsis.

### EFFECT OF THE INVENTION

According to the present invention, in vivo free radicals typified by superoxide anions in systemic organ or tissue injury caused by cerebral ischemia reperfusion injury, severe infection or sepsis can be promptly and quantitatively monitored, and whether the in vivo tissue conditions are good or not can be accurately diagnosed.

### BRIEF DESCRIPTION OF THE DRAWINGS

Fig. 1 is a drawing showing an outline according to one aspect of a device of the present invention;
Fig. 2 is an enlarged view of the vicinity of a tip end of a catheter according to one aspect of the device of the present invention;
Fig. 3 is a vertical sectional view cut alone line 3-3 in Fig. 1;
Fig. 4 is a drawing showing an outline of another aspect of the device of the present invention;

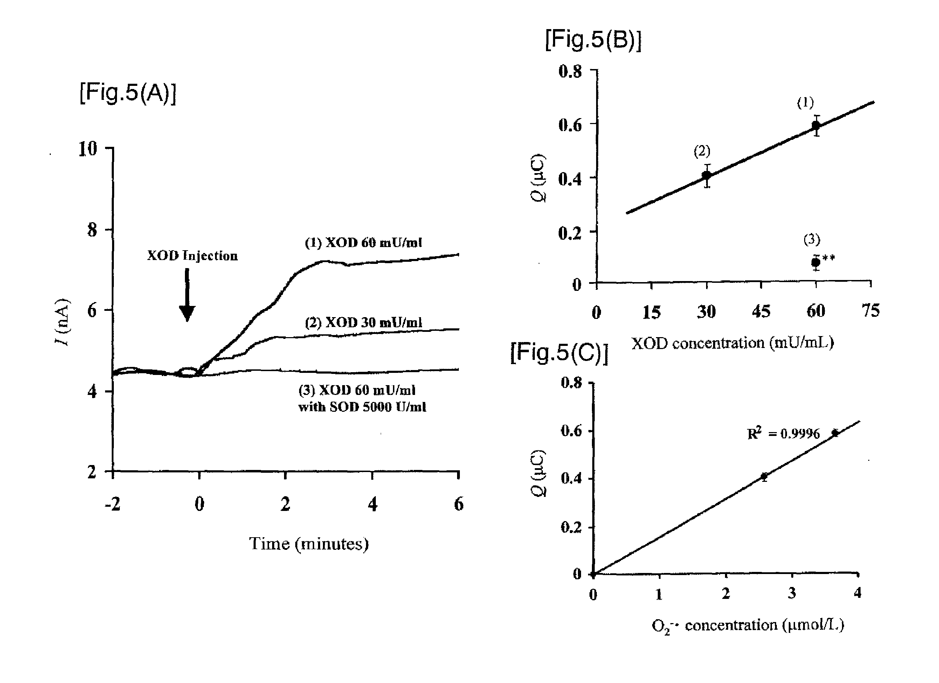

Fig. 5A is a diagram showing O₂⁻• currents (I, nA) generated by addition of two different amounts of xanthine oxidase with or without superoxide dismutase (SOD) to rat blood with xanthine, and the time course (min); the elevation of O₂⁻• current was revealed because of XOD dose-dependency and the addition of SOD diminished O₂⁻• current; the current data was measured two points per second, and smoothing procedures were applied to the data which contained noises and artifacts; Fig. 5B is a diagram showing the relationship between the quantity of electricity Q (µC) generated by addition of various amounts of XOD to rat blood with xanthine and the XOD concentration (mU/mL); the baseline of O₂⁻• current was defined as the stable state before XOD addition; the differences between the baseline and the current were integrated as the quantity of electricity (Q) at the time when the current reached plateau or peak; the elevation of the quantity of electricity was revealed because of XOD dose-dependency, and the addition of SOD attenuated the quantity of electricity generated by xanthine-XOD reaction; each value is expressed as mean ± standard deviation (SD) of 7 measurements; ** p < 0.01; Fig. 5C is a diagram showing the relationship between the quantity of electricity (Q) and O₂⁻• concentration in rat blood; the vertical axis indicates Q (µC), while the horizontal axis indicates O₂⁻• concentration (µmol/L); the O₂⁻• concentrations were calculated by the conventional method with xanthine/XOD reaction; the Q value increased lineally with the O₂⁻• concentration (p < 0.01); and each value is expressed as mean ± standard deviation (SD) of 7 measurements;
Fig. 6 is a drawing showing time table of the experiment;
Fig. 7 is a graph showing detection currents in the sham group and the SOD group; the graph of original raw detection currents contains noises and artifact, so that the graph has been smoothed by computer software in the drawing; after reperfusion, the detection currents in both groups began to rise up; after 20 min have passed after reperfusion, SOD (5mg/kg) was administered into the rats of the SOD group (★ mark in the figure); and the detection current in the control group kept rising up, while the detection current decreased in the SOD group;

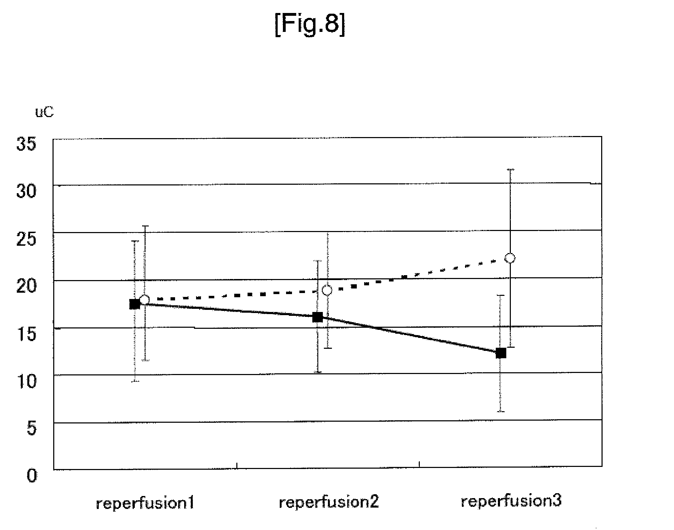

Fig. 8 is a graph showing detection currents of superoxide after reperfusion (mean ± SD); in the figure, the mark ○ and the dotted line indicate the detection current of the control group; the mark ■ and the solid line indicate the detection current of the SOD group; production of superoxide anion radicals was confirmed from the difference in detection current between the time of the baseline (in the stable state) and the reperfusion period; the integrated differences in each detection current is expressed as electrical charge (µC) in the graph; and in Reperfusion period 3 (40-60 min after reperfusion) the detection current in the SOD group significantly decreased, while the detection current in the control group kept increasing;

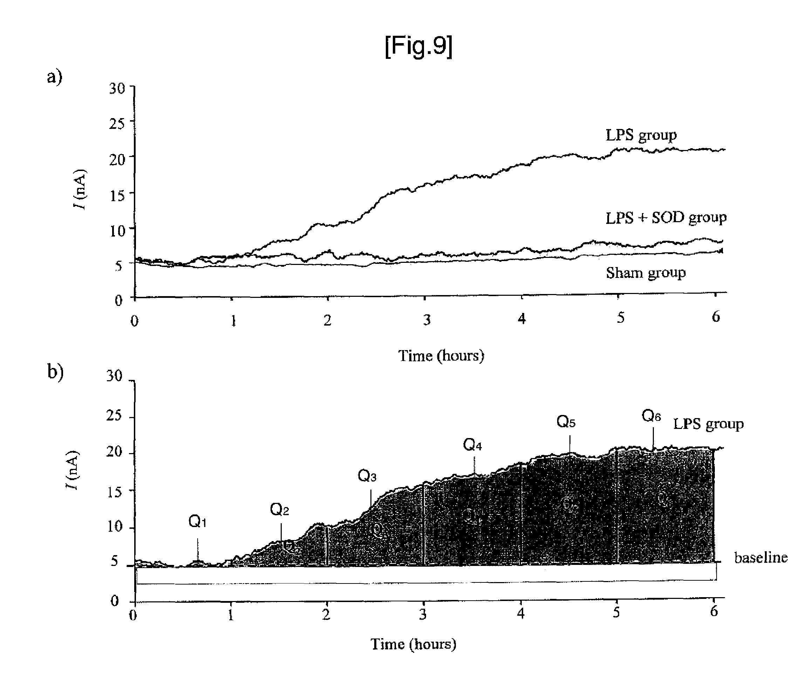

Fig. 9A is a diagram showing the relationship between the typified current I (nA) of superoxide anion radicals (O₂⁻•) of endotoxemic rats and the time course (hour); approximately, an hour after administration of lipopolysaccharide (LPS), O₂⁻• current began to increase and reached plateau at 5 hours in the LPS group; in the sham group, O₂⁻• current did not increase throughout the course; in the LPS + SOD group, the elevation of O₂⁻• current was attenuated by SOD administration; the current data was measured two points per second and smoothing procedures were applied to the data which contained noises and artifacts; Fig. 9B is a diagram showing the quantity of electricity (Q) produced which reflects the amount of the generated O₂⁻•; the baseline of O₂⁻• current is defined as the stable state before the LPS administration, and is indicated by the dotted line; the Q value was integration of the differences between the O₂⁻• current every hour after LPS administration and the baseline; and these gray areas indicate the hourly Q values;
Fig. 10 is a diagram showing the hourly quantity of electricity Q (µC) of superoxide anion radical in endotoxemic rats; after 2 hours, the Q values of LPS group (black bars) increased significantly compared to the sham group (white bars, p < 0.01); the Q values of the LPS + SOD group (gray bars) attenuated significantly compared to those of the LPS group (p < 0.01); and each value is expressed as mean ± standard deviation (SD) of 7 measurements, ** p < 0.01;
Fig. 11 is a diagram showing total plasma MDA levels (µM) and time course (hour) during the experiment; the total plasma MDA levels of the LPS group (black bars) and the total plasma MDA levels of the LPS + SOD group (gray bars) were significantly increased at 5 to 6 hours compared to those of the sham group (white bars, v. s. LPS group, p < 0.01, v. s. LPS + SOD group, p < 0.05); and each value is expressed as mean ± standard deviation (SD) of 7 measurements, *p < 0.05, ** p < 0.01;
Fig. 12 is a diagram showing plasma soluble intercellular adhesion molecule-1 (sICAM-1) levels (pg/ml) 6 hours after administration of lipopolysaccharide (LPS) in endotoxemic rats; the plasma sICAM-1 levels of the LPS group (black bars) and the plasma sICAM-1 levels of the LPS + SOD group (gray bars) increased significantly compared to those in the sham group (white bars, v. s. LPS and LPS + SOD groups, p < 0.01); and each value is expressed as mean ± standard deviation (SD) of 7 measurements, ** p < 0.01;

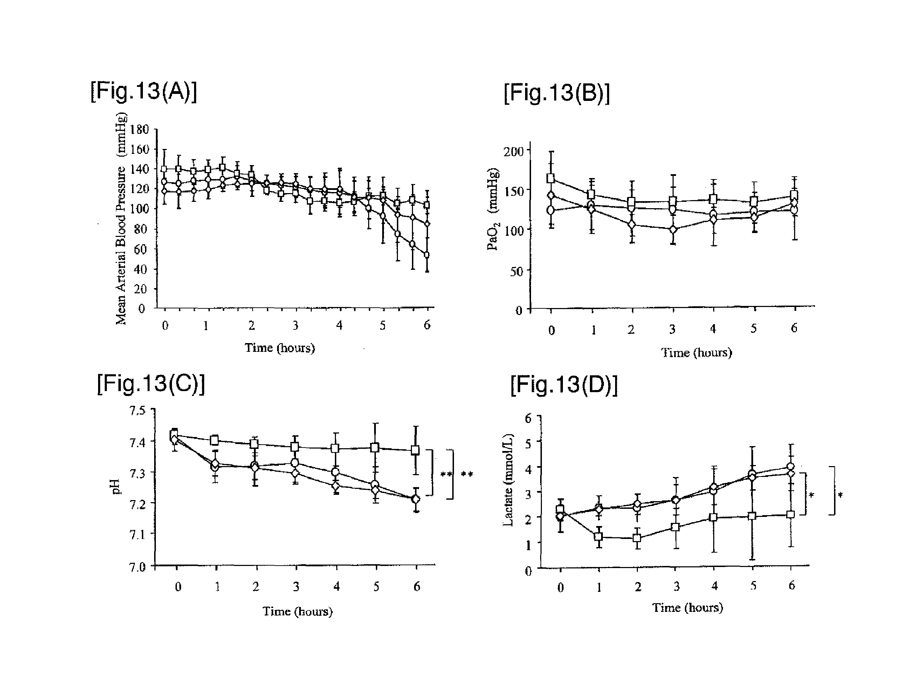

Fig. 13 shows parameters on mean arterial pressure (MAP) and arterial blood gas analysis during the experiment and the time course (hour) in each drawing, and the symbols of squares, circles and diamonds indicate the sham group, the LPS group and the LPS + SOD group, respectively; Fig. 13A is a diagram showing mean arterial pressure MAP (mmHg) during the experiment; there was no significant difference among the 3 groups; each value is expressed as mean ± standard deviation (SD) of 7 measurements; Fig. 13B is a diagram showing PaO₂ (mmHg) in the 3 groups; there was no significant difference among the 3 groups; Fig. 13C is a diagram showing pHs; pHs in the LPS group and the LPS + SOD group were significantly lower than those in the sham group (p < 0.01); Fig. 13D is a diagram showing lactate concentrations (mmol/L); the lactate concentrations in the LPS group and the LPS + SOD group was significantly lower than those in the sham group (p < 0.05); and each value is expressed as mean ± standard deviation (SD) of 7 measurements, *p < 0.05, ** p < 0.01;
Fig. 14 is a graph showing detection currents in the control and the ulinastatin groups, which shows the effect of ulinastatin in a forebrain ischemia reperfusion model;
Fig. 15 is a graph showing MDA levels in the brain tissue in the control and the ulinastatin groups, which shows the effect of ulinastatin in a forebrain ischemia reperfusion model;
Fig. 16 is a graph showing Plasma Total MDAs in the control and the ulinastatin groups, which shows the effect of ulinastatin in a forebrain ischemia reperfusion model;
Fig. 17 is a graph showing detection currents in the control group (Sham), the control group (Normothermia), the pre-ischemia hypothermia group (Pre-Hypothermia) and the post-ischemia hypothermia group (Post-Hypothermia), which shows the effect of hypothermia in a forebrain ischemia reperfusion model;
Fig. 18 is a graph showing MDA levels in the brain tissue in the sham group (Sham), the control group (Normothermia), the pre-ischemia hypothermia group (Pre-Hypothermia) and the post-ischemia hypothermia group (Post-Hypothermia), which shows the effect of hypothermia in a forebrain ischemia reperfusion model;
Fig. 19 is a graph showing Plasma Total MDAs in the sham group (Sham), the control group (Normothermia), the pre-ischemia hypothermia group (Pre-Hypothermia) and the post-ischemia hypothermia group (Post-Hypothermia), which shows the effect of hypothermia in a forebrain ischemia reperfusion model;
Fig. 20 is a diagram showing changes of blood sugar levels in the normal blood sugar group and the hyperglycemia group in a forebrain ischemia reperfusion model;
Fig. 21 is a graph showing detection currents in the normal blood sugar group and the hyperglycemia group, which shows the effect of hyperglycemia in a forebrain ischemia reperfusion model;
Fig. 22 is a graph showing MDA levels in the brain tissue in the normal blood sugar group and the hyperglycemia group, which shows the effect of hyperglycemia in a forebrain ischemia reperfusion model;
Fig. 23 is a graph showing Plasma Total MDAs in the normal blood sugar group and the hyperglycemia group, which shows the effect of hyperglycemia in a forebrain ischemia reperfusion model;
Fig. 24 is a diagram showing changes of PaO₂ in the normal oxygen group and the high-concentration oxygen group in a forebrain ischemia reperfusion model;
Fig. 25 is a graph showing detection currents in the normal oxygen group and the high-concentration oxygen group, which shows the effect of administration of high-concentration oxygen in a forebrain ischemia reperfusion model;
Fig. 26 is a graph showing MDA levels in the brain tissue in the normal oxygen group and the high-concentration oxygen group, which shows the effect of administration of high-concentration oxygen in a forebrain ischemia reperfusion model;
Fig. 27 is a graph showing Plasma Total MDAs in the normal oxygen group and the high-concentration oxygen group, which shows the effect of administration of high-concentration oxygen in a forebrain ischemia reperfusion model;
Fig. 28 is a graph showing detection currents in the control group, the high allopurinol group and the low allopurinol group, which shows the effect of administration of allopurinol in a forebrain ischemia reperfusion model;
Fig. 29 is a graph showing MDA levels in the brain tissue in the control group, the high allopurinol group and the low allopurinol group, which shows the effect of administration of allopurinol in a forebrain ischemia reperfusion model;
Fig. 30 is a graph showing Plasma Total MDAs in the control group, the high allopurinol group and the low allopurinol group, which shows the effect of administration of allopurinol in a forebrain ischemia reperfusion model;
Fig. 31 is a graph showing detection currents in the control group and the ulinastatin group, which shows the effect of ulinastatin in an endotoxinemia model;
Fig. 32 is a graph showing Plasma MDAs in the control group and the ulinastatin group, which shows the effect of ulinastatin in an endotoxinemia model;
Fig. 33 is a graph showing soluble ICAM-1s in the control group and the ulinastatin group, which shows the effect of ulinastatin in an endotoxinemia model;
Fig. 34 is a graph showing mean arterial pressures in the control group and the ulinastatin group, which shows the effect of ulinastatin in an endotoxinemia model; and
Fig. 35 shows parameters of arterial blood gas analysis and time course (hour) in each drawing, which shows the effect of ulinastatin in an endotoxinemia model; Fig. 35A is a diagram showing PaO₂ (mmHg); Fig. 35B is a diagram showing pH; Fig. 35C is a diagram showing base excess; and Fig. 13D is a diagram showing lactate concentration (mmol/L).

### DETAILED DESCRIPTION OF THE PREFERRED EMBODIMENT

The device for diagnosing tissue injury of the present invention (hereinafter referred to as "the device of the present invention") is configured by installing a superoxide anion radical sensor (referred to for short as "radical sensor") having a working electrode coated with polymeric iron porphyrin complex and at least a counter electrode at the tip end of a catheter which is insertable into the body.

In addition, in the device of the present invention, comparative means for comparing the concentration of superoxide anion radicals in blood measured by the radical sensor with a predetermined threshold to distinguish a tissue injury-based value and a healthy man-based value, is provided in, for example, a patient monitoring-system.

Furthermore, together with said radical sensor, a transfusion line for transfusing 1 or more drugs, and a sensor for obtaining other various information than the amount of superoxide anion radicals at the tip end of the catheter, e.g., a pressure transducer for measuring blood pressure or the like, are installed into the device of the present invention.

The various information obtained at the tip end of the catheter is sent as electric information to the patient monitoring-system, where diagnosis is made on whether the in vivo tissue conditions are good or not. If judged necessary, necessary drug solution is delivered through the transfusion line, so that cerebral ischemia reperfusion injury, as well as systemic organ or tissue injury caused by severe infection or sepsis is treated.

Hereinafter, the device of the present invention will be explained with reference to the drawings.

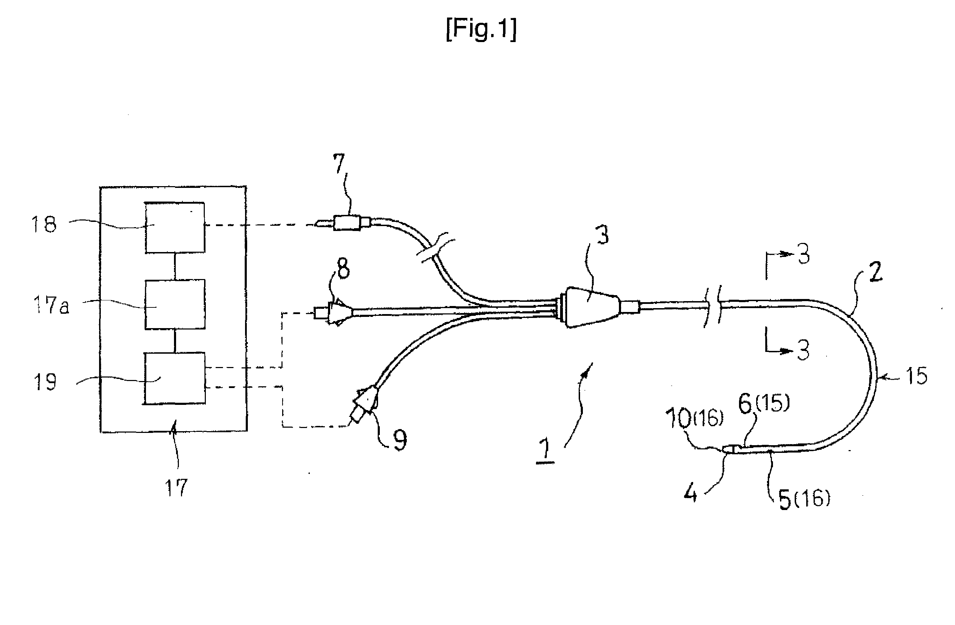

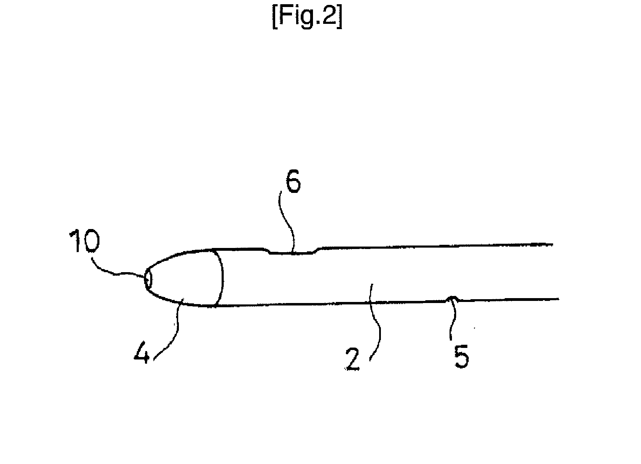

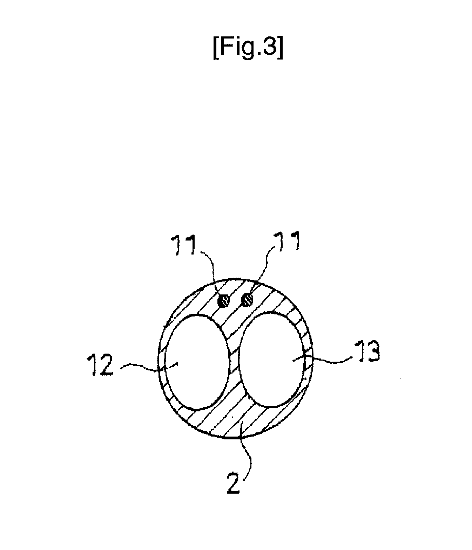

Fig. 1 is a schematic drawing showing one embodiment of the device of the present invention; Fig. 2 is an enlarged view of its tip end; and Fig. 3 is a vertical sectional view alone line 3-3 in Fig. 1.

A device for diagnosing tissue injury 1 according to the present embodiment is formed by a long catheter 2 insertable into the body, a radical sensor 15 provided inside of the catheter 2, and transfusion supply means 16. The catheter 2 has a basal portion 3 and a tip end 4. The radical sensor 15 is formed by a sensor electrode 6 capable of measuring superoxide anion radicals provided at the tip end 4 of the catheter 2, a lead wire connector 7 for a sensor provided at the basal portion 4 of the catheter 2, and a lead wire 11 for a sensor for connecting the sensor electrode 6 to the lead wire connector 7 for a sensor. The transfusion supply means 16 has at least one transfusion lines (2 transfusion lines in the present embodiment) 12, 13 provided at the inside of the catheter 2. One transfusion line 12 is communicated with a tip end hole 10 formed at the tip end member of the catheter 2 and capable of discharging transfusion from the tip end of the catheter 2, and the other transfusion line 13 is communicated with a side face hole 6 formed on the side face in the vicinity of the tip end of the catheter 2 and capable of discharging transfusion from the side face of the catheter 2. These transfusion lines 12, 13 are coupled to transfusion connectors 8, 9, respectively, which are provided at the basal portion 3 of the catheter 2.

When explained further, the catheter 2 used in the device of the present invention 1 has a diameter of such a size that the catheter 2 can be inserted into a thick blood vessel of a human body, for example, a diameter of approx. 2.4 to 4.0 mm. It is preferable that the main body of the catheter 2 is made of a biocompatible and flexible material, for example, polyurethane.

Furthermore, the transfusion lines 12, 13 provided in the catheter 2 are also preferably flexible tubes. The tubes are preferably made of polyurethane having a diameter of approx. 1.2 to 2.6 mm.

Furthermore, the lead wire 11 for a sensor need be a wire made of a material which assures an adequate amount of current. A metal wire made of silver, copper, platinum or the like is preferably used.

Furthermore, the electrode portion 6 used in the device of the present invention 1 is one which has been published by the inventors in Patent Literature 1, Non-patent Literature 5 or the like, and is made based on these literatures. In other words, the method of coating the working electrode with metal porphyrin complex polymerized coating, and the configuration of the counter electrode or the whole sensor can be readily obtained by referring to these.

For the sensor electrode 6, a needle-typed electrode invented early by the inventor may be used. Alternatively, this portion may be used only as the working electrode, and the catheter tip end 4 made of metal may be used as the counter electrode. Furthermore, individual electrodes may be coated with anticoagulant coating according to the art described in Patent Literature 2, whereby the electrodes can indwell within the blood vessel for a long period of time.

In the device of the present invention 1, on top of the above-described sensor electrode 6, the tip end hole 10 and the side face hole 5 for transfusion are provided. These holes 5, 10 are respectively connected to the transfusion lines 12, 13 for discharging liquid drug through these holes, as necessary.

Meanwhile, the connector for 7 a radical sensor and the connectors 8, 9 for transfusion are provided at the basal portion 3 of the catheter 2 of the device of the present invention 1.

Among these, the connector 7 for a radical sensor which forms the radical sensor 15 is coupled to judgment means 18 provided in a patient monitoring-system 17 which measures an ever-changing amount of superoxide anion radicals in blood of a patient and diagnose whether the in vivo tissue conditions are good or not in a disease such as cerebral ischemia reperfusion injury.

Furthermore, the connectors 8 and 9 for transfusion which form the transfusion supply means 16 are coupled to a transfusion device 19 provided in the patient monitoring-system 17 which delivers liquid drug in the required amount to the transfusion lines 12 and/or 13 based on a command from a control portion 17a provided in the patient monitoring-system 17. Furthermore, in some cases, the transfusion lines 12 and/or 13 may be used as the pressure transducer, and may be used for example for measurement of central venous pressure.

The operation of the present embodiment will now be explained.

The reason why it is possible to diagnose whether the in vivo tissue conditions are good or not in cerebral ischemia reperfusion injury, and systemic organ or tissue injury caused by severe infection or sepsis, etc. using the device of the present invention 1 will be as descried below.

That is, the amount of active superoxide anion radicals is significantly increased in blood of a patient who has received these injuries. The metal porphyrin complex polymerized coating used in the device of the present invention 1 can catalyze the dismutation reaction of superoxide anion radicals and detect the oxidation current depending on its concentration. Therefore, it is possible to measure the amount of active superoxide anion radicals on real time. Particularly, it is possible to measure superoxide anion radicals with the radical sensor 15 more reliably by inserting the catheter 2 into a proper measurement portion of superoxide anion radicals in the living body, for example, into the venous or artery portion including the jugular venous portion, carotid artery portion, etc., the internal organ portion including heart, or the like.

Next, cerebral ischemia reperfusion injury can easily be diagnosed by the judgment means 18, for example, by predetermining a threshold to distinguish a patient with cerebral ischemia reperfusion injury and a healthy man based on the amount of active superoxide anion radicals of a patient conventionally and clinically diagnosed as cerebral ischemia reperfusion injury. In the same manner, systemic organ-tissue injury caused by severe infection or sepsis can be easily diagnosed by measuring the amount of active superoxide anion radicals of a patient clinically diagnosed as systemic organ-tissue injury caused by severe infection-sepsis and setting a threshold based on this.

Furthermore, worsening or improvement of the tissue injury with a time course can be diagnosed and drug in an adequate amount can be injected at appropriate timing by keeping indwelling the catheter 2 portion of the device of the present invention 1 in the body.

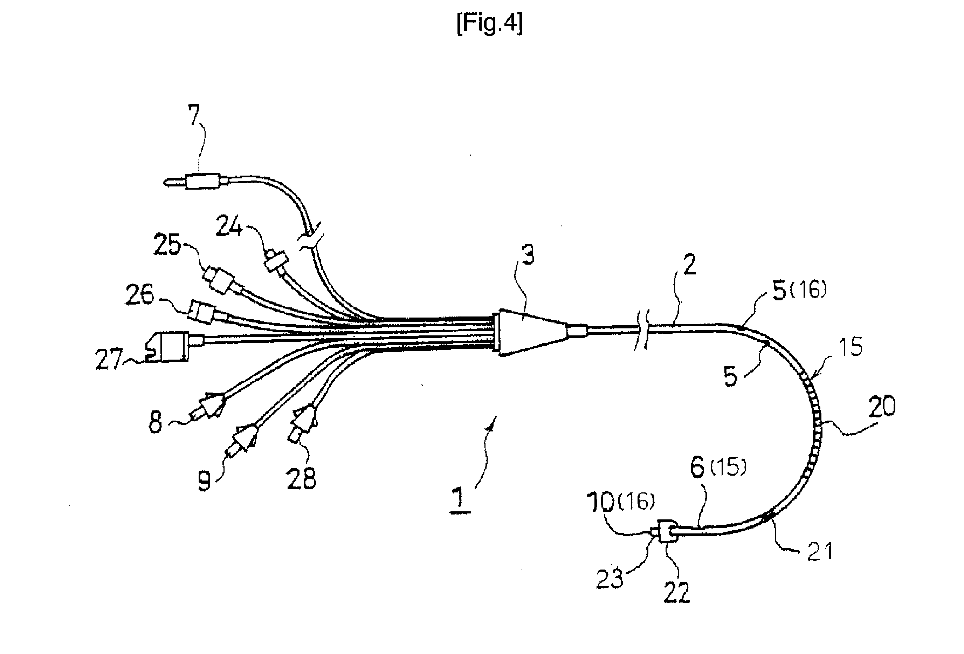

Another embodiment of the device of the present invention 1 will now be explained with reference to Fig. 4 which is a schematic drawing.

In the present embodiment, a thermal filament 20 and a thermistor 21 are provided at the front side of the tip end 4 of the catheter 2, and a balloon 22 and an optical fiber 23 are provided at the tip end 4 of the catheter 2. A balloon expansion valve 24, a thermistor connector 25, a thermal filament connector 26, an optical module connector 27, a connector 28 for transfusion are connected individually to the basal portion 3 of the catheter 2. The same numerals are assigned to the same portions as those in the above-described embodiment.

The inventive device 1 of this embodiment includes substantially all configuration requirements of the device according to the embodiment shown in Fig. 1, and further includes necessary sensors for diagnosis, that is, the thermal filament 20, the thermistor 21 and the optical fiber 23. Accordingly, corresponding to these, the thermistor connector 25, the thermal connector 26, and the optical module connector 27 are also provided at the basal portion 3 of the catheter 2. These sensors enable diagnosis at an enhanced level.

Furthermore, a balloon 22 is installed in the device of the present invention 1 of the above-described embodiment. It is also possible to inflate the balloon 22 with air supplied from the valve expansion valve 24 so as to be of use for treatment for heart failure or the like.

### EXAMPLES

It will now be verified that diagnosis for whether the in vivo tissue conditions are good or not, that is, for tissue injury is feasible by measuring superoxide anion radicals in rats using the device of the present invention 1.

Handling of rats as a model of the living body in the Examples was approved by Animal Experiment Committee of Yamaguchi University, and all rats were treated in accordance with the guideline by National Institutes of Health, USA.

Furthermore, all data were expressed as mean ± standard deviation (SD) in statistic analysis in these Examples. Statistic significance between the 2 groups was determined by one-way analysis of variance (ANOVA). Data were analyzed using a statistics package program SPSS 10.0 (SPSS Inc., Chicago, IL, USA). A value P < 0.05 was judged as statistically significant.

### < Example 1 >

In vitro validation of radical sensor in rat blood

The radical sensor 15 was verified in rat blood before it was applied to an in vivo model.

That is, the amount of superoxide anion radicals was measured by immersing the sensor electrode 6 formed at the tip end 4 of the catheter 1 of the device of the present invention 1 in rat blood collected from a rat (see Fig. 5).

### 1) Subject rats

Twenty-eight male specific pathogen free Wister rats, weighing 250-300 g, were used. The rats were anesthetized by 4% isoflurane and 96% oxygen. After laparotomy, whole blood was sampled from the inferior vena cava with 500 U of heparin. Blood samples were used as soon as possible.

### 2) Preparation and measurement of rat blood

Individual bloods were randomly assigned to one of three experimental groups: 30 mU/ml of xanthine (hereinafter referred to as "XAN") + xanthine oxidase (hereinafter referred to as "XOD") (n = 7, low XOD group) (see (2) in Fig. 5), 60 mU/ml of XAN + XOD (n = 7, high XOD group) (see (1) in Fig. 5), and 60 mU/ml of XAN + XOD + 5000 units/ml of superoxide dismutase (hereinafter referred to as "SOD", from bovine erythrocytes, Sigma Chemical, St. Louis, MO, USA) (n = 7, XOD + SOD group) (see (3) in Fig. 5).

Five milliliters of blood was stirred and incubated at 37 °C. XAN was added to blood at a final concentration of 150 µM SOD was added at the final concentration of 5000 units/ml only in the XOD + SOD group. The radical sensor 15 of the device of the present invention 1 was inserted into blood, and O₂⁻•current was measured continuously. After stabilization of the current, XOD was added to the blood. The final concentrations of XOD were 30 mU/ml in the low XOD group and 60 mU/ml in the high XOD and the XOD + SOD groups, respectively. The O₂⁻• current (I (nA)) was measured for 360 seconds in rat blood after XOD addition. The current data was measured two points per second and smoothing procedures were applied to the data which contained noises and artifacts. The baseline of the O₂⁻• current was defined as the stable state before XOD addition. The differences of current between the baseline (at just before XOD) and the post-XOD levels were integrated as the quantity of electricity (Q) until the point where the current reached plateau or peak.

### 3) Measurement result

In Fig. 5A, results of measurement for the O₂⁻• current (I (nA)) based on the quantity of superoxide anion radicals generated in the blood and their time courses were indicated in each of the 3 groups shown in (1) to (3).

Consequently, the elevation of O₂⁻• current was revealed because of XOD dose-dependency, and the addition of SOD diminished O₂⁻• current.

The O₂⁻• current (I) generated by the oxidation of xanthine could be caught by the O₂⁻• sensor in rat blood as well as in saline, because the addition of SOD diminished the O₂⁻• current (Fig. 5A).

The oxidation of xanthine at that time was according to the following formula (A):

xanthine + 2O₂ + H₂O -> urate + 2O₂⁻• + 2H⁺; k₁ (A)

This reaction was catalyzed by XOD.

In Fig. 5B, the generated O₂⁻• was evaluated for the rat blood in the 3 groups (1) to (3) shown in Fig. 5A, by the quantity of electricity (Q) of O₂⁻•, which reflected the amount of the generated O₂⁻•, because the change of the O₂⁻• current was variable in vivo. Specifically, the base line of the current was defined as the stable state before the XOD addition. The differences of current between before and after XOD addition were integrated as Q value (µC) during the time when the current reached plateau or peak. Each value is expressed as mean ± standard deviation (SD) of 7 measurements, ** p < 0.01.

Consequently, the Q value was revealed because of XOD dose-dependency in rat blood (r² = 0.9897, y = 0.0066x + 0.2155, see (1) in Fig. 5B and (2) in Fig. 5B). Furthermore, the Q value of 60 mU/ml of XOD was significantly attenuated by addition of 5000 U/ml of SOD (p < 0.01, see (3) in Fig. 5B).

In Fig. 5C, the relationship between the quantity of electricity (Q) and the O₂⁻• concentration in rat blood is shown. The vertical axis indicates Q (µC), while the horizontal axis indicates the O₂⁻• concentration (µmol/L).

O₂⁻• concentration was calculated by the conventional method with xanthine/XOD reaction.

Specifically, a conventional method for estimation of the O₂⁻• concentration as a function of XOD activity has been established, based on the steady-state approximation for the rate of the above-described formula (A) and dismutation of O₂⁻• according to the following formula (B).

2O₂•⁻ + 2H⁺ -> H₂O₂ + O₂; k₂ (B)

The differential equation for O₂⁻• concentration (d[O₂⁻•/dt = k₁[XOD]? k₂[O₂⁻•]²) suggests that the O₂⁻• concentration under steady-state conditions is proportional to the square root of the XOD concentration. With use of the known values of k₁ and k₂, one can relate Q value directly to the O₂⁻• concentration.

Fig. 5C obtained as a result of this shows that there was significant correlation between the Q value and the O₂⁻• concentration (r² = 0.9952, p < 0.01). The Q value increased linearly with the O₂⁻• concentration. In the human peripheral blood, the same results were obtained as those in the rat blood.

It is found from the results shown in Figs. 5A to 5C that the Q value would be an appropriate indicator to evaluate the amount of O₂⁻• generated in vivo. Furthermore, the xanthine/XOD reaction can be used for sensor calibration in rat blood, if it is necessary.

### < Example 2 >

Measurement of superoxide concentration in an acute phase ischemic disease model (rats)

### 1) Subject rats

Sixteen male Wister rats (260-280 g) were randomly assigned to 2 groups, i.e., the reperfusion group (n = 8) and the reperfusion with SOD group (n = 8). There is no statistical difference of body weight of rats between the 2 groups.

### 2) Configuration of the radical sensor 15

The radical sensor 15 installed in the device of the present invention is a catheter-typed one shown in Fig. 1. An electrodeposited film of a polymeric iron porphyrin derivative attached to a carbon electrode is placed in stainless steel tube as an auxiliary counter electrode.

### 3) Experimental method

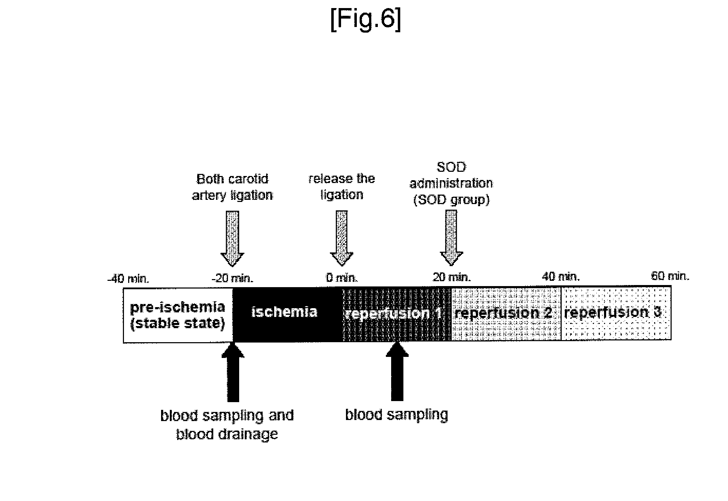

A dummy probe and a guide tube of microdialysis were embedded in the brain parenchyma of the rat on the previous day of the experiment under pentobarbital anesthesia (intraperitoneal administration, 50 mg/kg). The guide tube was fixed by dental resin at 3 mm outside and 3 mm anterior of the bregma of the right hemisphere. Under isoflurane anesthetization and mechanical ventilation through a tracheostomized tube, the electrochemical superoxide sensor was inserted into left internal jugular vein through the thyroid vein and the other venous branches were ligated. The microdialysis probe (C-I-4-02, Eicom Corporation, Kyoto, Japan) was replaced with the dummy probe to start microdialysis at 3 µl/min. Rats of forebrain ischemia model were made by bilateral common carotid artery occlusion and shedding blood for 20 min to get the systolic blood pressure between 40-45 mmHg. The forebrain ischemia was confirmed by the electroencephalogram and the elevation of glutamate concentration in the microdialysate (Busto et al.). Reperfusion was achieved after the bilateral forebrain ischemia by releasing the bilateral carotid artery occlusion and returning the shedding blood. Blood pressure was maintained by saline or hydroxyethyl starch transfusion for 60 min after the reperfusion. SOD (5mg/kg) was administered intravenously at 20 min after the reperfusion in the reperfusion with SOD group. The glutamate concentration of perfusate of microdialysis was measured by high performance liquid chromatography with an electrochemical detector (Eicom Corporation, Kyoto, Japan). Perfusate was collected for 20 min each for pre-ischemia, during ischemia and post-ischemia 1, 2, and 3 (Fig. 6). Excess superoxide was evaluated from the difference of detection currents by the radical sensor 15 between pre-ischemia and post-ischemia. PO₂, PCO₂, pH and base excess of arterial blood, arterial pressure, pharyngeal and rectal temperature were also measured during the experiment. At the end of the experiment, the rat was euthanized by intravenous injection of pentobarbital (50 mg/kg) and KCL. After the euthanasia, the brain was taken out and the position of microdialysis probe was confirmed.

### 4) Result

There was no significant difference with the volume of the shed blood volume, rectal and pharyngeal temperature, and blood pressure in pre-ischemia, during ischemia and post-ischemia between the 2 groups (Table 1). Though acidosis was observed in both groups by ischemia and reperfusion, there was no significant difference between the 2 groups (Table 1). In both groups the concentration of glutamate in the perfusate of microdialysis rose up during ischemia and fell down by reperfusion (Table 2). Such a rise of the glutamate concentration represents the forebrain ischemia, for which significant difference was not seen between the 2 groups (Table 2).

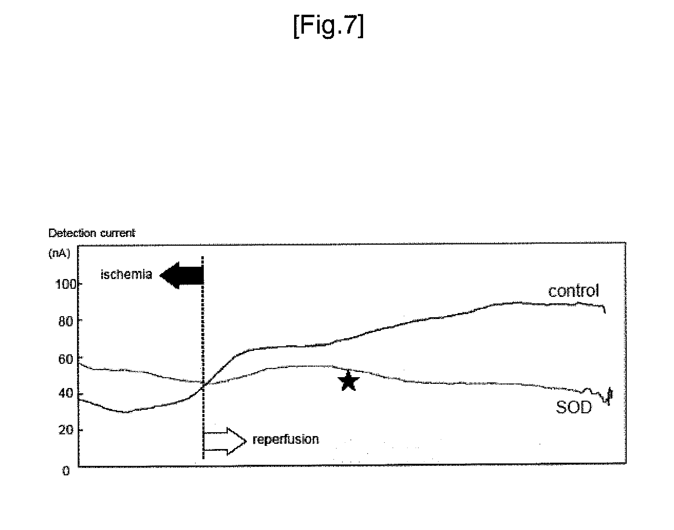

The base line of the detection current of the radical sensor 15 was defined as the stable state before ischemia and the differences were measured during ischemia-reperfusion. The detection current contained noises and artifacts, so that it was determined by smoothing the graph by a moving-average method using computer software (Fig. 7). The detection current was integrated for 20 min each as electrical charge. After the reperfusion, the detection currents increased from the baseline by the production of superoxide in the 2 groups. At 20 min after the reperfusion, SOD (5mg/kg, with a star mark) was intravenously administered. Although the detection current increased further in the reperfusion group, it began to decrease just after the administration of SOD in the reperfusion with SOD group. The detection current decreased further during the reperfusion period 3 (40-60 min after the reperfusion) and the statistically significant difference was observed between the 2 groups (p < 0.05, Table 3 and Fig. 8).

**[Table 1]**

| | | | | | | |
|---|---|---|---|---|---|---|
| Table 1. Physiological parameters and shed blood volume in the rats. | | | | | | |

| | | Reperfusion group (n=8) | | Reperfusion with SOD group (n=8) | | |
|---|---|---|---|---|---|---|
| Body weight(g) | | 265 | ±8.4 | 259 | ±11.4 | n.s. |
| Shed blood volume(ml) | | 6.3 | ±1.14 | 5.5 | ±1.22 | n.s. |
| Body temperature(rectal,) | pre-ischemia | 36.6 | ±0.5 | 37.0 | ±0.3 | n.s. |
| | ischemia | 36.9 | ±0.1 | 36.8 | ±0.9 | n.s. |
| | reperfusion | 37.2 | ±1.0 | 37.1 | ±0.1 | n.s. |
| Body temperature (pharyngeal) | pre-ischemia | 37.0 | ±0.8 | 37.0 | ±0.7 | n.s. |
| | ischemia | 35.6 | ±2.1 | 36.0 | ±0.9 | n.s. |
| | reperfusion | 36.9 | ±0.6 | 37.1 | ±0.4 | n.s. |
| Blood pressure(mmHg) | pre-ischemia | 171.8 | ±14.1 | 156.2 | ±19.6 | n.s. |
| | ischemia | 48.3 | ±5.9 | 46.0 | ±3.1 | n.s. |
| | reperfusion | 87.5 | ±19.8 | 98.2 | ±25.0 | n.s. |
| PH | pre-ischemia | 7.28 | ±0.05 | 7.29 | ±0.06 | n.s. |
| | reperfusion | 6.90 | ±0.05 | 6.91 | ±0.04 | n.s. |
| Base Excess | pre-ischemia | -9.38 | ±3.56 | -6.9 | ±2.94 | n.s. |
| | reperfusion | -24.5 | ±2.35 | -25.2 | ±2.20 | n.s. |

Reperfusion herein represents forebrain ischemia-reperfusion for 20 min. Values are mean ± standard deviation (SD).
There was no significant difference between the 2 groups.

**[Table 2]**

| | | | | | |
|---|---|---|---|---|---|
| Table 2. Glutamate concentrations in microdialyate. | | | | | |

| | pre-ischemia | ischemia | reperfusion1 | reperfusion2 | reperfusion3 |
|---|---|---|---|---|---|
| Reperfusion group | 0.9±0.85 | 10.2±4.50 | 8.5±6.91 | 1.6±2.16 | 2.8±5.94 |
| Reperfusion with SOD group | 2.1±2.30 | 9.8±11.6 | 3.4±1.46 | 2.2±1.20 | 2.1±1.57 |

Microdialysate was collected every 20 min. Glutamate increased by ischemia and fell down after reperfusion. There is no significant difference between the 2 groups.

**[Table 3]**

| | | | |
|---|---|---|---|
| Table 3. Integrated superoxide current during reperfusion periods in the rats of forebrain ischemia rats. | | | |

| | reperfusion 1 | reperfusion 2 | reperfusion 3 |
|---|---|---|---|
| Reperfusion group | 17.9 ± 6.3 | 18.8 ± 6.2 | 22.1 ± 9.4 |
| Reperfusion with SOD group | 17.5 ± 8.2 | 16.1 ± 5.9 | 12.1 ± 6.2 |
| | n.s. | n.s. | p<0.05 |

Values are expressed as mean ± standard deviation (SD), and P < 0.05 was considered statistically significant. Integrated detection current expresses electrical charge (µC) of superoxide for 20 min each. The detection current of reperfusion group kept rising up after reperfusion, while those of reperfusion with SOD group attenuated in reperfusion period 3. There was significant difference between the 2 groups in reperfusion period 3.

### 5) Discussion

Most ROS in the living body derive from superoxide, which gives an important meaning to quantify superoxide on real time in the living body. As described before, individual conventional measurement devices have their own drawbacks. The radical sensor 15 of the device of the present invention 1 uses polymeric iron porphyrin derivative as a cytochrome c mimic for the sensor electrode 6 and it has high selectivity of superoxide, which enables reliable measurement of superoxide. The surface of the electrode is tough chemically and mechanically enough to stand use in the living body.

Superoxide becomes extinct naturally by dismutation [2O₂⁻ + 2H⁺ -> O₂ + H₂O₂ (H₂O₂: hydrogen peroxide)]. Polymeric iron porphyrin derivative (material of the sensor electrode 6) which has the same structure as 6th coordination position of the heme in the active center of cytochrome c can catalyze the dismutation of superoxide and the oxidation current can be detected. The linear correlation between the amount of superoxide and the detection current has been confirmed by the experiment of xanthine oxidase and xanthine oxide reaction which generates superoxide anion radicals. It was also confirmed that the detection current of superoxide decreased promptly by administration of SOD and that the electrode can detect only superoxide selectively as it does not detect hydrogen peroxide. Therefore, this superoxide selective electrode with an amplifying device and memory was used.

The forebrain ischemia leads to energy failure and rapid neuronal necrosis. In the study related to the present invention, ischemia of neurons was represented by an increase of glutamate concentration in the perfusate of microdialysis (Table 2). During ischemia, glutamate concentration increased to 4.7 to 11.3 folds and the elevation of glutamate declined to the pre-ischemia levels.

After reperfusion, there is influx of inflammatory cells and oxygen that lead to excess ROS production. In the study related to the present invention, superoxide current gradually increased at least for 60 min after reperfusion in the reperfusion group (Figs. 7 and 8, Table 3). Biomembrane structure contains many unsaturated double bonds which are especially sensitive to free radical-induced lipid peroxidation. Excess ROS can directly damage mitochondria membranes of neurons by the result of lipid peroxidation and spread the damage of the ischemic brain.

The study related to the present invention showed that intravenous administration of SOD at 20 min after reperfusion could decrease the forebrain ischemia/reperfusion induced superoxide in the jugular vein (Fig. 7, Fig. 8, and Table 3). There are some reports that SOD over expression or pretreatment of antioxidants can show some neuroprotection in ischemia-reperfusion animal model. The results of the study related to the present invention might closely relate to these results that SOD or antioxidants might reduce free radicals such as superoxide and inhibit lipid peroxidation of neurons. Cherubini et al. reported remarkable reduction of SOD and antioxidants in patients with severe ischemic stroke. In these cases, superoxide might increase in the brain and the internal jugular vein. In that situation, the superoxide-sensitive sensor electrode 6 of the device of the present invention 1 might be a very useful clinical monitor at the bed side. Free radical scavengers such as Edaravone have been used clinically for ischemic stroke patients in Japan. Therefore, it becomes more important to evaluate ROS behavior in terms of pathophysiology of stroke and of an effect of radical scavengers or antioxidants at bed side in human. The in vivo needle-typed or catheter-typed radical sensor 15 developed by the inventor, Yuasa et al. can be easily inserted into the living body and can detect superoxide. When the long term sensitivity and stability of the radical sensor are resolved, behavior of free radicals such as superoxide will be revealed and the biosensor might be in clinical use.

As described above, excess production of superoxide can be detected by the radical sensor inserted in vivo into forebrain ischemia of rats according to the device of the present invention 1. In addition, rapid reduction of superoxide by SOD administration can also be detected by the radical sensor. On the other hand, superoxide increased further after reperfusion without SOD. The in vitro real-time measurement of superoxide of the device of the present invention 1 can reveal a part of behavior of superoxide in acute phase ischemic disease.

### < Example 3 >

Measurement of superoxide concentration in acute phase sepsis model (in rats)

### 1) Subject rats

Twenty-one male specific pathogen free Wister rats, weighing 250-300 g, were used in this example. The rats were randomly assigned to one of the 3 groups, i.e.; sham group: intravenous bolus and continuous administration of saline (n = 7); LPS group: bolus lipopolysaccharide (hereinafter referred to as "LPS") and continuous administration of saline (n = 7); and bolus LPS and continuous administration of superoxide dismutase (SOD) (n = 7).

### 2) Configuration of the radical sensor 15

The radical sensor 15 installed in the device of the present invention 1 is a catheter-typed one shown in Fig. 1. An electrodeposited film of polymeric iron porphyrin derivative attached to a carbon electrode is placed in stainless steel tube as an auxiliary counter electrode.

### 3) Experiment method

The rats were anesthetized by 3% isoflurane and 97% oxygen. They were mechanically ventilated through a tracheostomized tube. An arterial catheter was inserted to measure blood pressure and to take a blood sample in the left femoral artery. A venous catheter was inserted to administer drugs from the left femoral vein. The tip end of the radical sensor 15 installed in the tip end 4 of the catheter 2 of the device of the present invention 1 was inserted from the right external jugular vein to the right atrium. After the surgical operation, anesthesia was changed to 0.9% isoflurane and 60% N2 in oxygen. Thereafter, the O₂⁻• current was measured continuously. Pancuronium bromide (0.2 mg every one hour) was given intravenously for mechanical ventilation and heparin (100 units i.v., every one hour) was given to prevent coagulation around the radical sensor 15.

After the stabilization of the blood gases and the O2-• current, LPS derived from Escherichia coli O111: B4 (weighing 3µg/g, Sigma Chemical, St. Louis, MO, USA), in the LPS group and the LPS + SOD group, or an equivalent volume of saline in the sham group, were given intravenously. In the LPS + SOD group, after one hour from LPS administration, SOD was given intravenously (25 units/g in 1µl/g of saline and continuous infusion at the rate of 25 units/g/hr, Sigma Chemical, St. Louis, MO, USA). The same dose of saline was given in the LPS and sham groups. The mean blood pressure was measured continuously and recorded every 20 min. Blood sampling (0.5ml) and arterial blood gas analysis was performed once an hour.

O₂⁻• measurement lasted for 6 hours after the LPS administration. The baseline of the O₂⁻• current was defined as the stable state before the LPS administration. The difference of the O₂⁻• current between the baseline and the current level in each group was integrated every hour as the quantity of current (Q) (Fig. 9B).

### 4) Result

The typified O₂⁻• currents measured by the device of the present invention 1 in acute phase sepsis model rats, a type of endotoxemic rats, are shown in Fig. 9A. At an hour after lipopolysaccharide (LPS) administration, the O₂⁻• current began to increase and reached plateau at 5 hours in the LPS group. In the sham group, the O₂⁻• current did not change during the course. In the LPS + SOD group, O₂⁻• generation was suppressed by the SOD administration, so that the O₂⁻• current was inhibited.

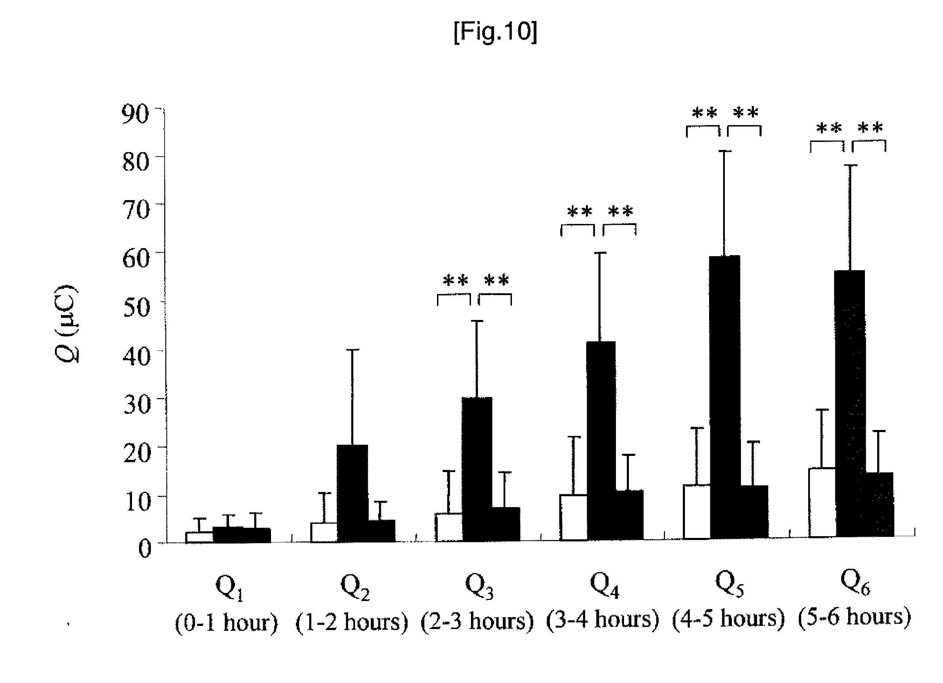

In Fig. 9B, how to calculate the Q values in endotoxemic rats is shown. The base line of the current was defined as the stable state before LPS administration. The differences between the baseline and the O₂⁻• current were integrated as Q value each hour after LPS administration. The Q values were significantly increased in the LPS group after 2-3 hours to 5-6 hours compared to both the sham group and the LPS + SOD group (Fig. 10, p < 0.01).

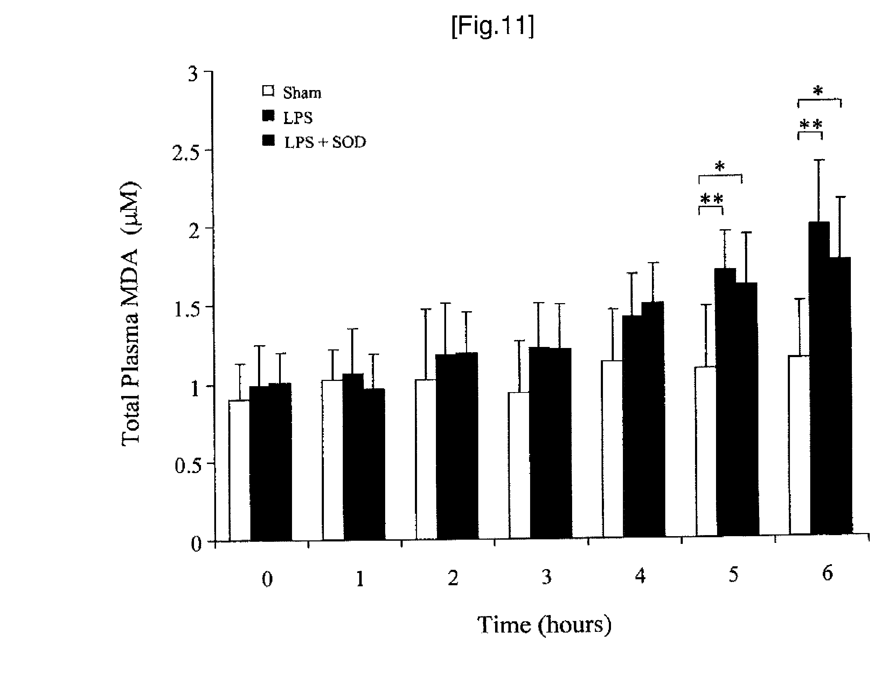

Total plasma malondialdehyde (MDA) levels were measured to evaluate degree of lipid peroxidation every hour after LPS administration in the 3 groups (Fig. 11). Two hours after LPS administration, total plasma MDA levels in the LPS group and the LPS + SOD group tended to increase compared to that in the sham group. After 5 hours, total plasma MDA levels in the LPS group and the LPS + SOD group were significantly higher than that in the sham group (p < 0.01 v. s. LPS group, p < 0.05 v. s. LPS + SOD group). Significant differences of MDA levels were not seen between the LPS group and the LPS + SOD group.

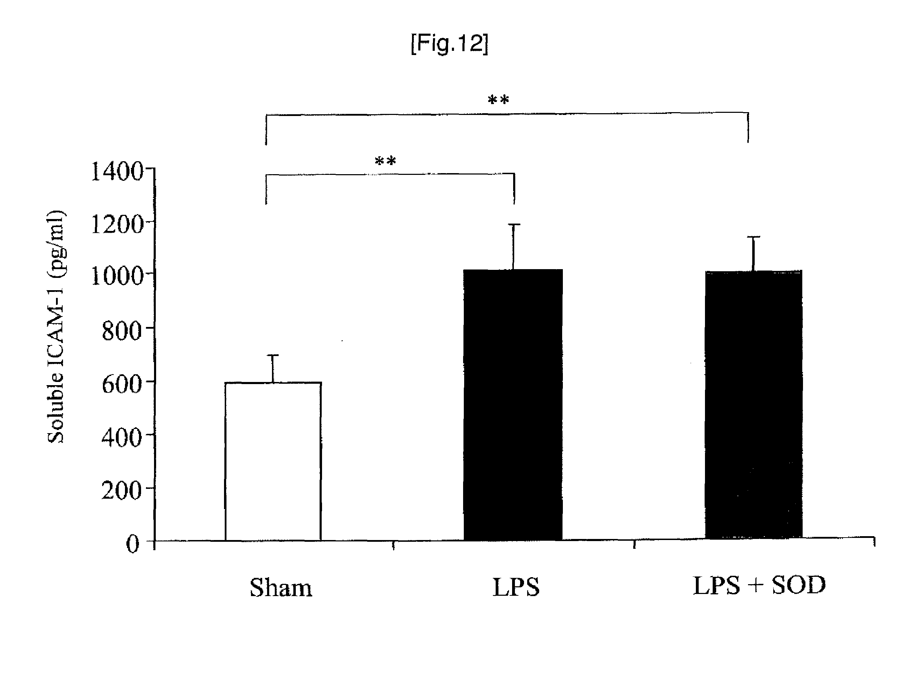

Plasma soluble intercellular adhesion molecule-1 (sICAM-1) levels (pg/ml) were measured to make evaluation in the 3 groups (Fig. 12). Plasma sICAM-1 levels at 6 hours after LPS administration herein were analyzed by Quantikine® Rat sICAM-1 (CD54) Immunoassay Kit (R&D System, Inc., Minneapolis, USA). The plasma sICAM-1 levels in the LPS group and the plasma sICAM-1 levels in the LPS + SOD group increased significantly compared to those in the sham group (as shown by white bars, p < 0.01 v. s. LPS group and LPS + SOD group).

Temporal changes of mean arterial pressure (MAP) (mmHg), PaO₂ (mmHg), pH and lactate concentration (mmol/L) during the experiment were individually measured (see Fig. 13A to D). The symbols of squares, circles and diamonds in the figure indicate the sham group, the LPS group and the LPS + SOD group, respectively. Here, blood samples were taken from the femoral arterial catheter once an hour and plasma was stored at - 80°C until analysis. Total plasma MDA levels were analyzed by BIOXYTECH® MDA-586 kit (OxisResearch^{™}, Foster, CA, USA). The method was based on the reaction of chromogenic reagent, N-methyl-2-phenylindole with MDA at 45°C. In addition, arterial blood gas and lactate were analyzed with the ABL System 555 (Radiometer Medical A/S), Copenhagen, Denmark). Mean arterial pressure (MAP) of the LPS group tended to be lower than the other groups after 4 hours, but a significant difference was not shown during the course (Fig. 13A). There was no difference in PaO₂ among the 3 groups during the course (Fig. 13B), while pH in the LPS group and the LPS + SOD group were significantly lower than that in the sham group (Fig. 13C). Lactate concentrations in the LPS group and the LPS + SOD group were significantly higher than those in the sham group (Fig. 13D).

### 5) Discussion

In the LPS group, O₂⁻• current began to increase 1 hour after the LPS administration and reached plateau at 5 hours (Fig. 9A). In the LPS + SOD group, continuous administration of SOD attenuated the O₂⁻• current, which proved that the radical sensor 15 responded in vivo (Fig. 9A). The Q values were applied to evaluate O₂⁻• generation in vivo, because the Q values reflect a periodic amount of O₂⁻• generation in the device of the present invention 1 as shown in Fig. 5C. The Q values in the LPS group increased significantly after 2-3 hours through 5-6 hours compared to those in other groups (Fig. 10). Therefore, the Q values were applicable to evaluate the generated O₂⁻• in vivo.

In the state of endotoxemia caused by acute phase sepsis, various cells such as activated neutrophils, macrophages, endothelial cells, and others generate O₂⁻•. The possible sources were considered to include mitochondrial respiratory chain, metabolic cascade of arachidonic acid, xanthine oxidase and NADPH oxidase. This radical sensor 15 can react to the O₂⁻• generated by any of these reactions, and the values might relate to the activities of immune cells or enzymes which catalyze O₂⁻• generation in the LPS group compared to that in the sham group (Figs. 9A and 10).

It is speculated that the generated O₂⁻• would generate more potent toxic free radicals, such as peroxynitrite (ONOO⁻) and hydroxyl radical (OH⁻•), which enhance lipid peroxidation and injure various tissues. MDA, the index of lipid peroxidation in plasma, began to increase after 2 hours in the PS group compared to that in the sham group, there were significant differences after 5 hours between the LPS group and the sham group (Fig. 11). This fact coincided with the elevation of the Q value in the LPS group (Fig. 10). Furthermore, ROS generated in circulating blood caused lipid peroxidation of endothelium, so that endothelium injury and microcirculatory disorder were distinguished. Consequently, hypotension and metabolic acidosis induced by high level of soluble intercellular adhesion molecule (sICAM-1) in plasma (Fig. 12) and high concentrations of lactate persisted (Fig. 13). Therefore, it is important to recognize the production of O₂⁻• as shown in the present study (Figs. 9A and 10) and those O₂⁻• values that might be a predictive factor of tissue injury and a target of treatment in various diseases.

In the LPS + SOD group, SOD did not improve MDA, sICAM-1 or physiologic parameters in the present study (Figs. 11 to 13). It has been reported that high-dose SOD enhanced lipid peroxidation. SOD, one of O₂⁻• scavengers, catalyzes O₂⁻• and water to hydrogen peroxide (H₂O₂) and changes to the one of the most potent radicals, i.e., OH⁻•, by Fenton reaction. This might be the reason that the administration of SOD did not improve lipid peroxidation, endothelium injury, hypotension or metabolic acidosis (Figs. 11 to 13), although the O₂⁻• current and the Q value were suppressed in the LPS + SOD group (Figs. 9A and 10).

Although conventional methods such as cytochrome c reduction assay, nitroblue tetrazolium reduction assay and chemiluminescent detection can catch whole O₂⁻• generated in each system, these methods do not give us real time data in vivo. The radical sensor 15 installed in the device of the present invention 1 can catch O₂⁻• on real time although it cannot detect the whole production of O₂⁻•.

Therefore, the radical sensor 15 installed in the device of the present invention 1 is the only way to detect O₂⁻• in vivo at present. In the study related to the present invention, the tip end of the radical sensor 15 was placed in the right atrium of rats, so that the changes of O₂⁻• reflected O₂⁻• generation in the whole body.

The accuracy and usefulness of the device of the present invention 1 was confirmed by dose-dependency of xanthine/XOD reaction in human blood. The Q value in the human peripheral blood also increased dose-dependently with the O₂⁻• generated by xanthine/XOD reaction, which indicates the Q value in human blood. These results suggested that this novel method of O₂⁻• monitoring and the evaluation would be a very useful tool to understand ROS-related pathophysiological state.

In addition, it is important to evaluate degree of activation of immune cells such as neutrophils by ROS quantification, because neutrophil elastase inhibitors such as sivelestat are used for clinical treatment for lung injury caused by sepsis. The needle-shaped radical sensor 15 installed in the device of the present invention 1 which is developed by the inventors can be easily inserted into the living body, and superoxide can be detected. It is anticipated that trend of in vivo ROS can be confirmed more accurately by further studies related to the long term use, clinical trials, or the like of the sensor.

In conclusion, the device of the present invention 1 is the first device we are aware of that can monitor and evaluate O₂⁻• generated in vivo directly and continuously.

### < Example 4 >

Effects of ulinastatin on superoxide generation in a forebrain ischemia reperfusion model

### 1) Experimental conditions

Fourteen male Wister rats (250-300 g) were randomly assigned to the control group and the ulinastatin group. Under isoflurane anesthetization and mechanical ventilation, the superoxide sensor was inserted into the internal jugular vein, and superoxide values were measured continuously. Forebrain ischemia was made by bilateral common carotid artery occlusion-hemorrhagic hypotension. After ischemia was maintained for 20 min reperfusion was performed, and the brain tissue and serum was sampled at 60 min after reperfusion. 5000 units/kg of ulinastatin was intravenously administered just after reperfusion in the ulinastatin group, while the equal amount of saline was administered in the control group.

### 2) Result

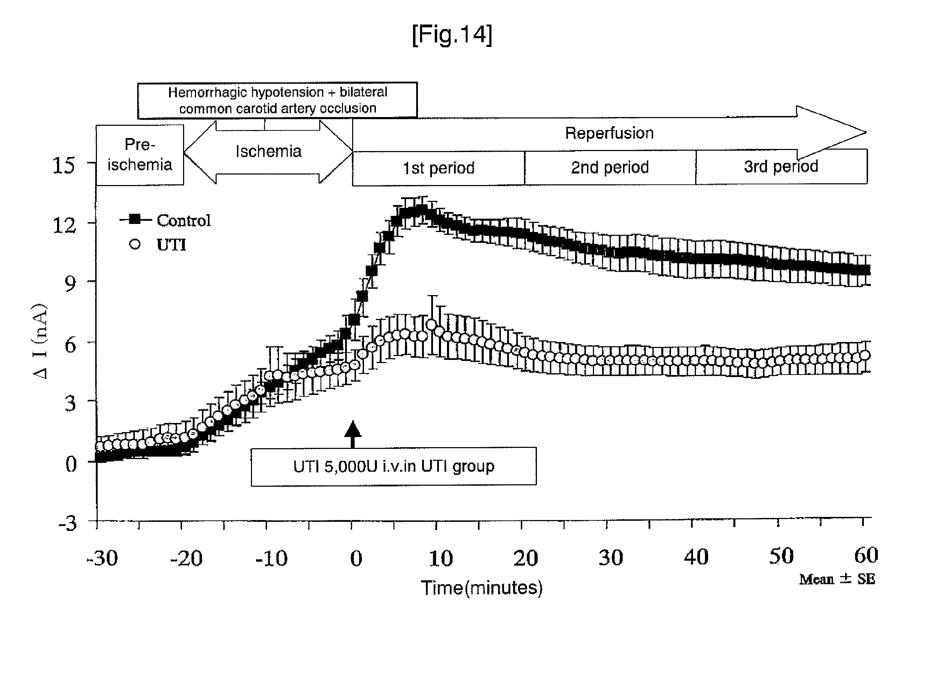

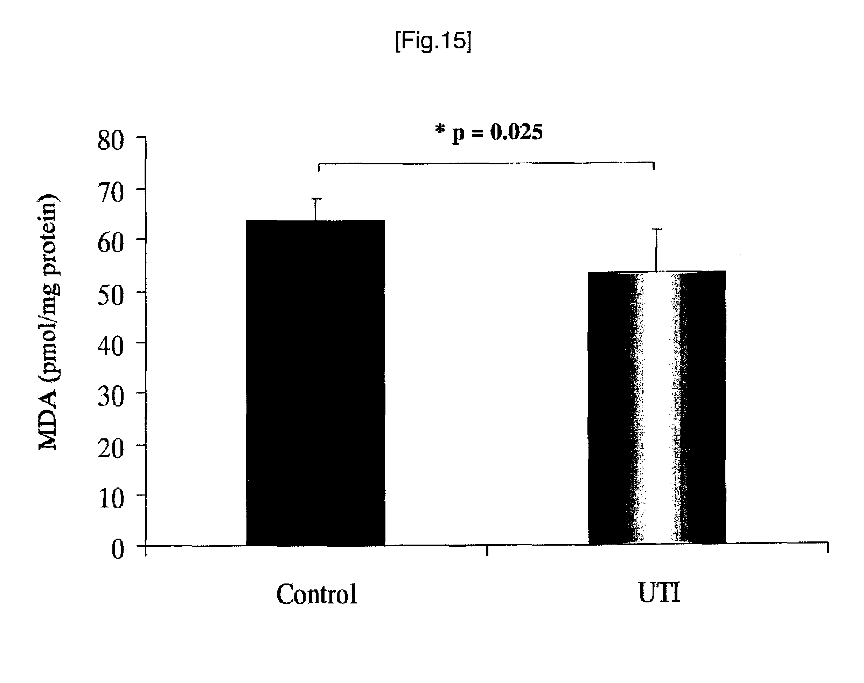

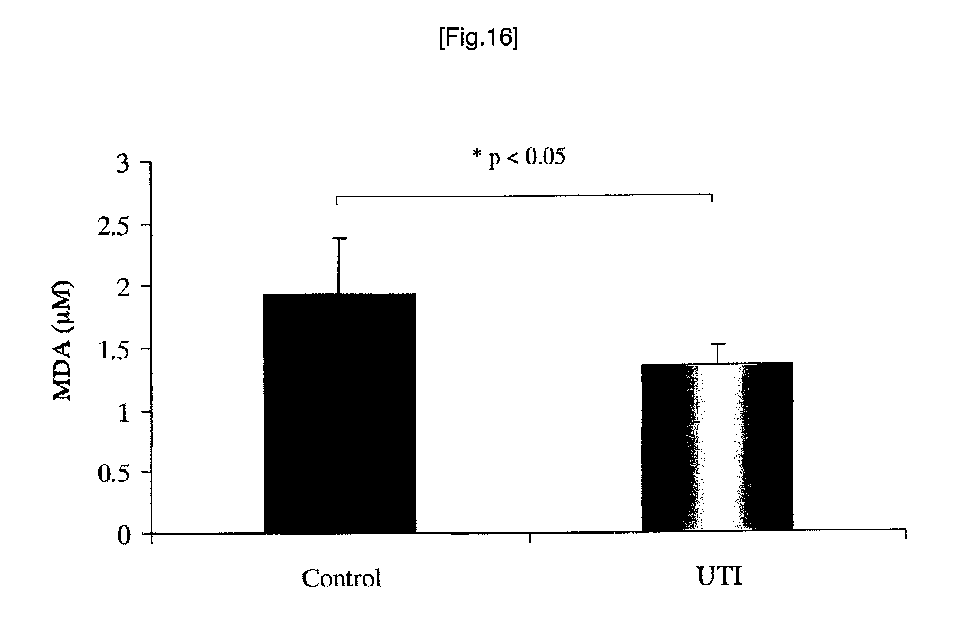

In the ulinastatin group, the O2-• current values after the reperfusion were significantly suppressed (Fig. 14). In addition, malondialdehyde (MDA), which is a lipid peroxidative substance in the brain tissue and serum, was also significantly suppressed in the ulinastatin group (Figs. 15, 16).

### 3) Significance

Superoxide generation as well as generation of lipid peroxidative substance MDA were suppressed by ulinastatin, which is protease inhibitor. Ulinastatin can control oxidation stress when cerebral ischemia reperfusion injury occurs, and thus can be a treatment drug for stroke and hypoxic encephalopathy.

### < Example 5 >

Effect of hypothermia on superoxide generation in a forebrain ischemia reperfusion model

### 1) Experimental conditions

Twenty-eight male Wister rats (250-300 g) were randomly assigned to the sham group, the control group, the pre-ischemia hypothermia group, and the post-ischemia hypothermia group. Under isoflurane anesthetization and mechanical ventilation, the radical sensor 15 installed in the device of the present invention 1 was inserted into the internal jugular vein, and superoxide values were measured continuously. Forebrain ischemia was made by bilateral common carotid artery occlusion-hemorrhagic hypotension. After ischemia was maintained for 10 min, reperfusion was conducted, the brain tissue and serum was sampled at 120 min after reperfusion. Body temperatures (pharyngeal) were kept at 37.0 °C during the course in the sham and the control groups. Furthermore, in the sham group, only hemorrhagic hypotension without bilateral common carotid artery occlusion was not conducted during operation of ischemia. In the pre-ischemia hypothermia group, the body temperatures were kept at 34 °C during the period from pre-ischemia until 120 min after reperfusion. In the post-ischemia hypothermia group, the body temperatures were kept at 34 °C for the period from just after reperfusion until 120 min after reperfusion.

### 2) Result

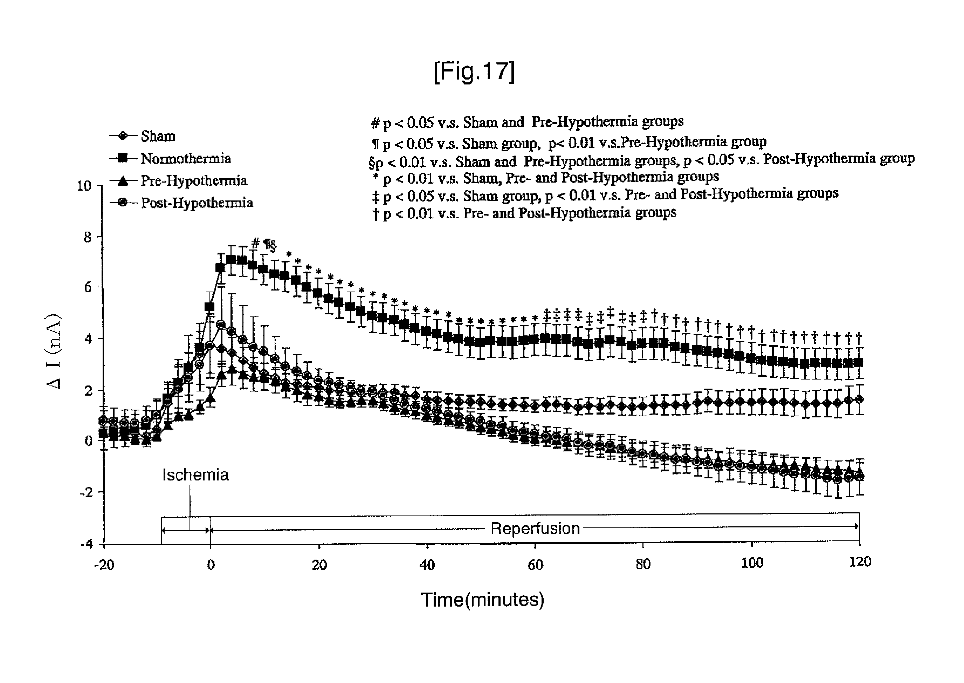

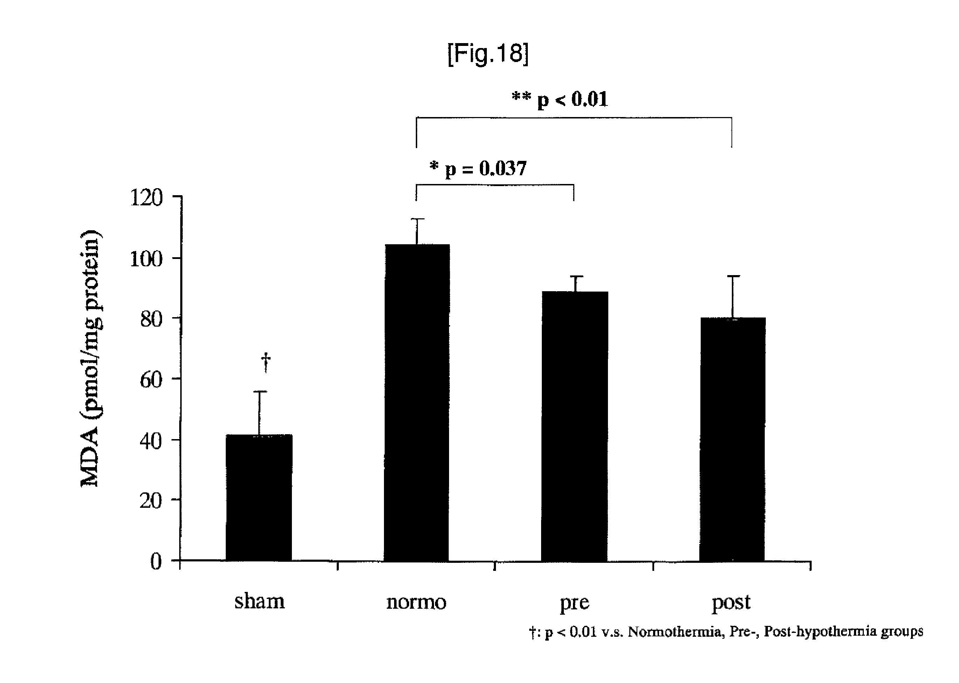

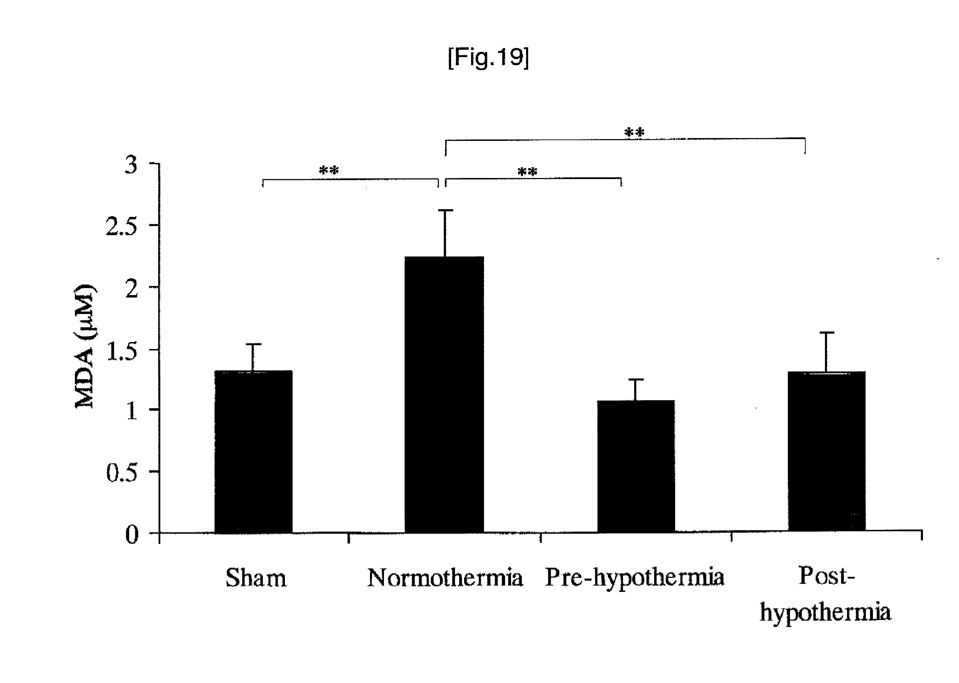

In the sham group, the O₂⁻• current values were significantly lower than those in the control group during the course. The O₂⁻• current values during ischemia and reperfusion were significantly suppressed in the pre-ischemia hypothermia group compared to those in the control group. The O₂⁻• current values after reperfusion were significantly suppressed in the post-ischemia hypothermia group compared to those in the control group (Fig. 17). Furthermore, MDAs in the brain tissue and in serum were significantly suppressed in the 2 groups, i.e., the pre-ischemia group and the post-ischemia hypothermia group compared to those in the control group (Figs. 18, 19).

### 3) Significance

Usefulness of hypothermia in acute brain injury is being demonstrated, while there is no data on hypothermia's suppression on superoxide generation. It is possible to use superoxide values as a target hypothermia therapy in the future.

### < Example 6 >

Effect of hyperglycemia on superoxide generation in a forebrain ischemia reperfusion model

### 1) Experimental conditions

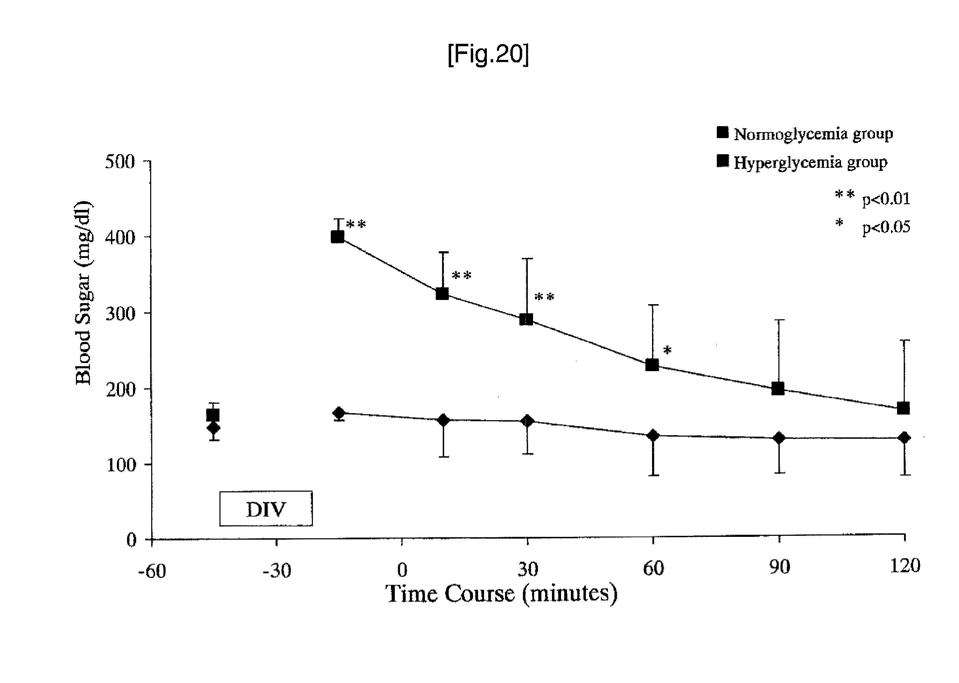

Fourteen male Wister rats (250-300 g) were randomly assigned to the normal blood sugar group and the hyperglycemia group (Fig. 20). Under isoflurane anesthetization and mechanical ventilation, the superoxide sensor was inserted into the internal jugular vein, and superoxide values were measured continuously. Forebrain ischemia was made by bilateral common carotid artery occlusion-hemorrhagic hypotension. After ischemia was maintained for 10 min, reperfusion was conducted and the brain tissue and serum was sampled at 120 min after reperfusion. In the hyperglycemia group, hyperglycemia state was created by intravenous administration of 10 µl/g of 20% dextrose solution before creating ischemia. The equal amount of saline was administered in the normal blood sugar group.

### 2) Result

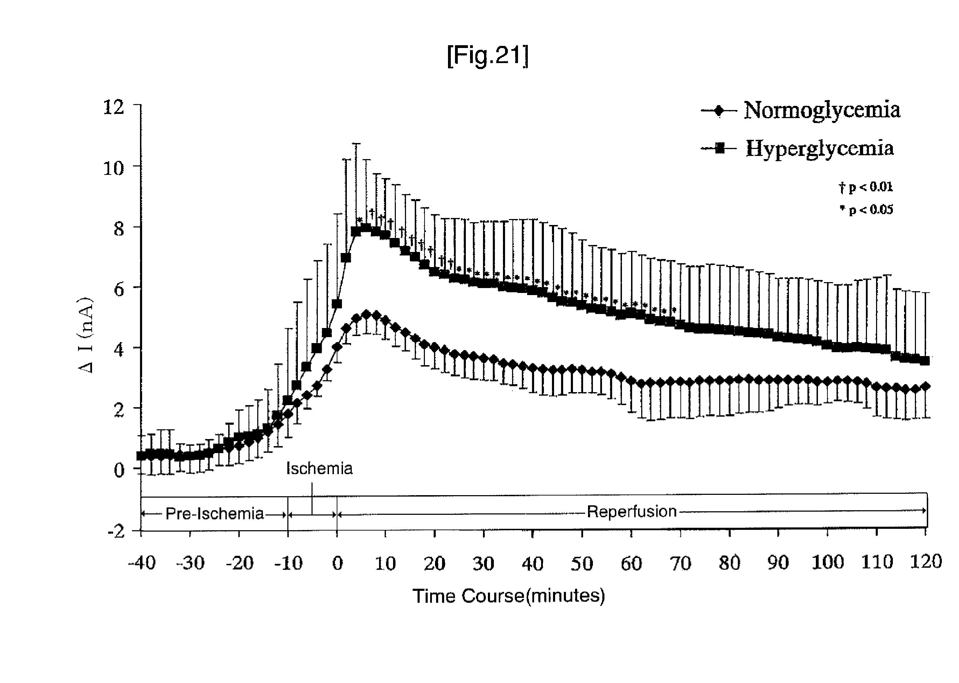

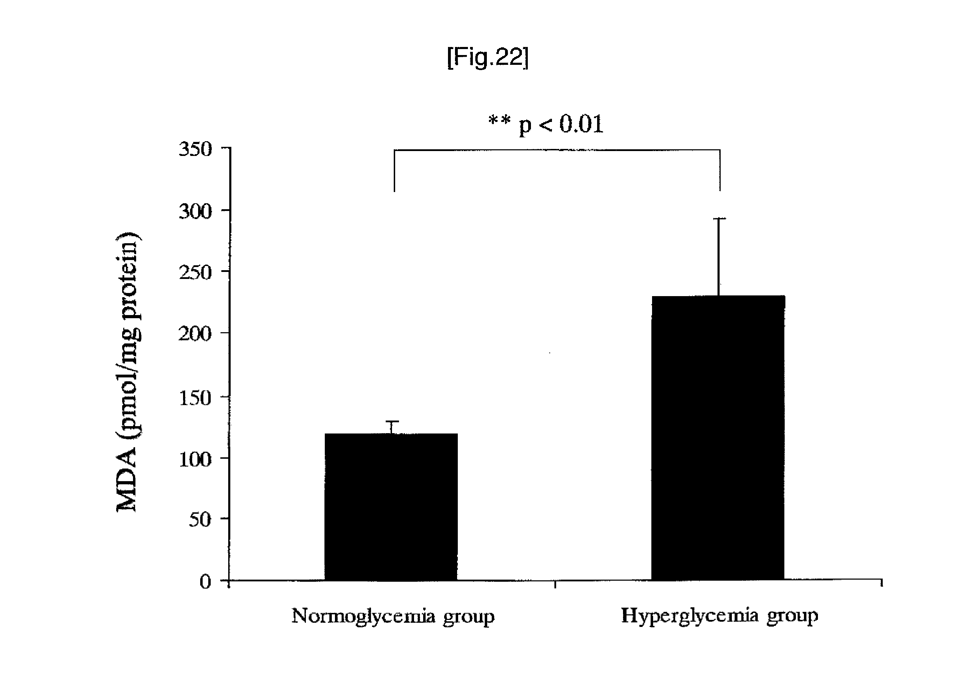

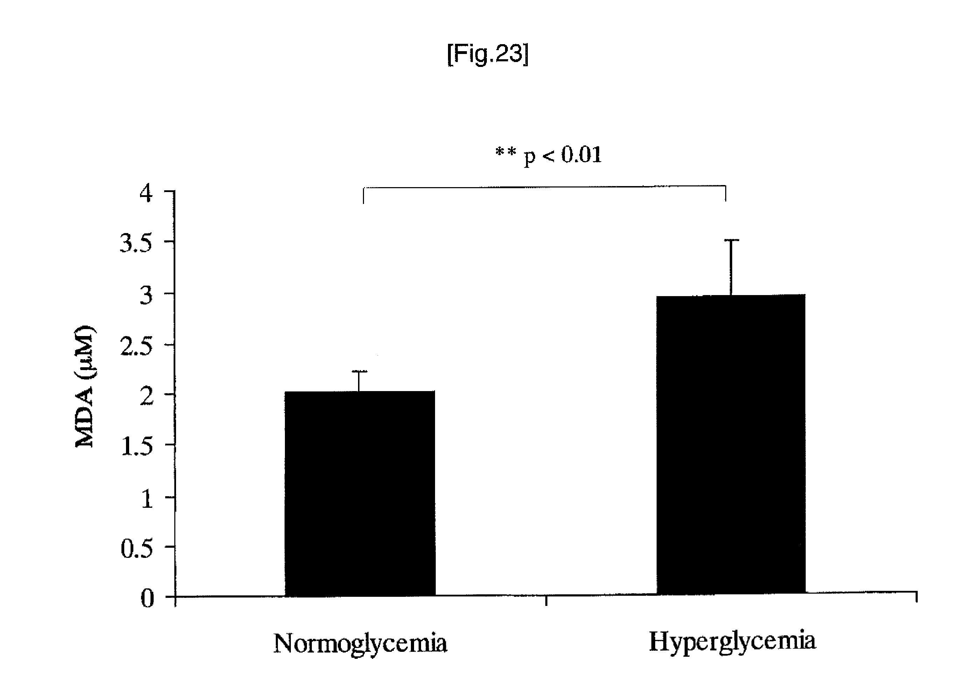

In the hyperglycemia group, the O₂⁻• current values during the course were significantly higher than those in the normal blood sugar group (Fig. 21). MDAs were also significantly increased in the brain tissue and in serum in the hyperglycemia group (Figs. 22, 23).

### 3) Significance

It was revealed that superoxide is involved in pathological conditions of acute brain injury caused by short-term hyperglycemia. It was indicated that hyperglycemia exacerbates oxidation stress, and it was suggested that glycemic control is very important in the whole body management in severe patients.

### < Example 7 >

Effect of administration of high-concentration oxygen on superoxide generation in a forebrain ischemia reperfusion model

### 1) Experimental conditions

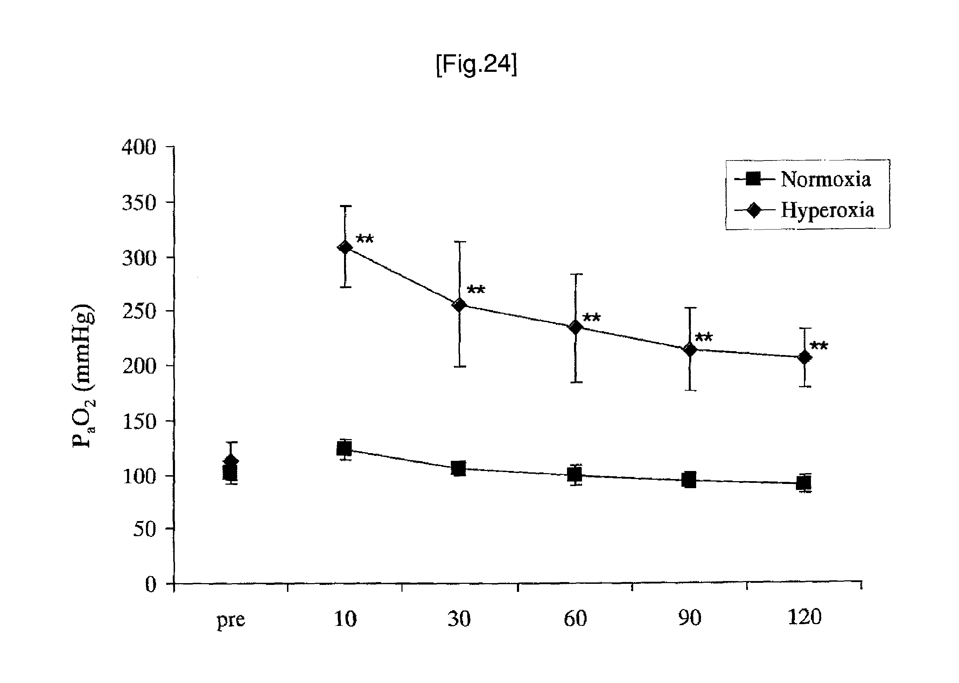

Fourteen male Wister rats (250-300 g) were randomly assigned to the normal oxygen group and the high-concentration oxygen group (Fig. 24). Under isoflurane anesthetization and mechanical ventilation, the superoxide sensor was inserted into the internal jugular vein, and superoxide values were measured continuously. Forebrain ischemia was made by bilateral common carotid artery occlusion-hemorrhagic hypotension. After ischemia was maintained for 10 min, reperfusion was conducted, and the brain tissue and serum were sampled at 120 min after reperfusion. In the normal oxygen group, inhalant oxygen concentration during the course was maintained at 40%. In the high-concentration oxygen group, the inhalant oxygen concentration was maintained at 40% up until during ischemia, changed to 100% just after reperfusion, and maintained until 120 min after reperfusion.

### 2) Result

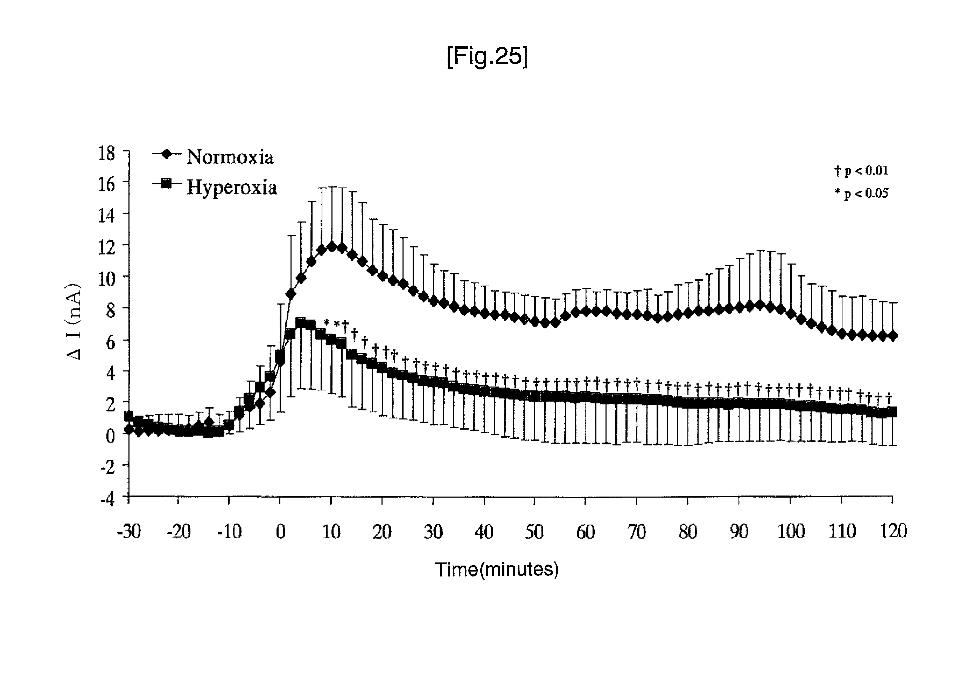

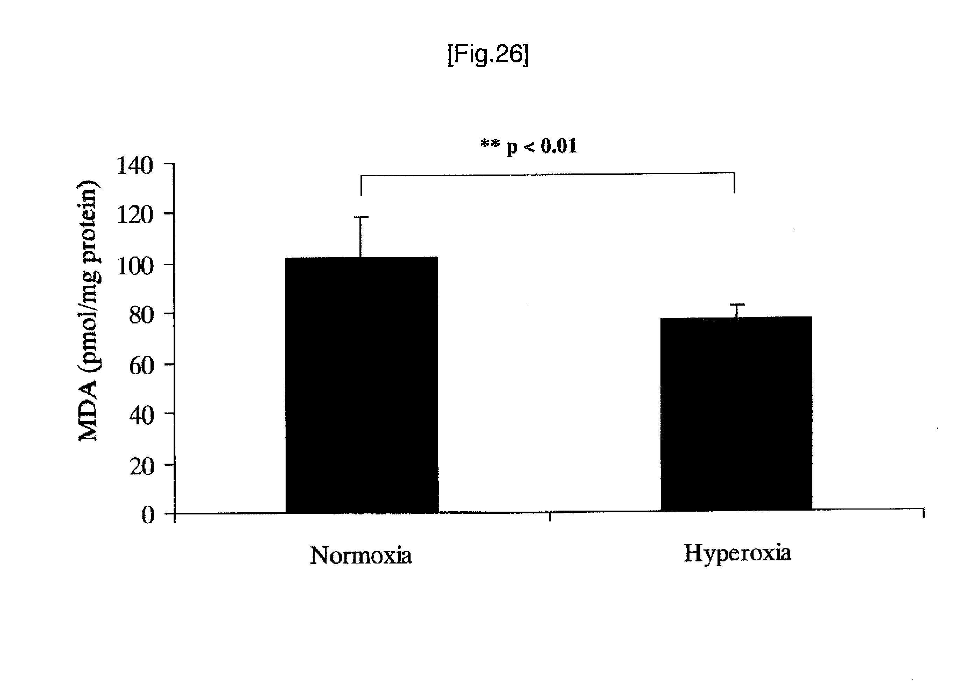

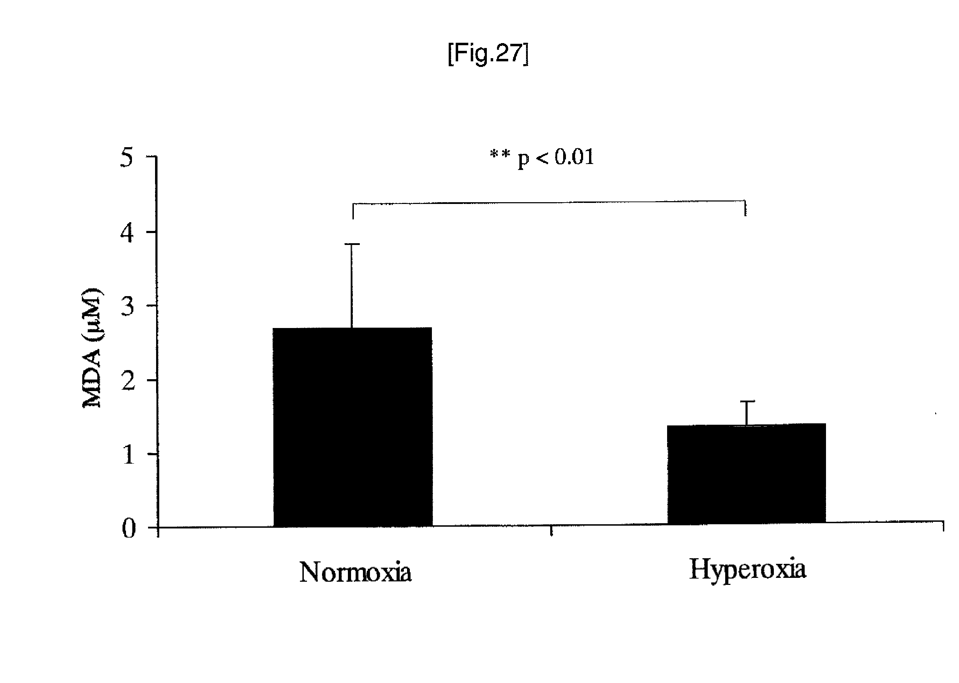

In the high-concentration oxygen group, superoxide values during the course were significantly suppressed compared to those in the normal oxygen group (Fig. 25). MDAs in the brain tissue and in serum were also significantly suppressed in the high-concentration oxygen group (Figs. 26, 27).

### 3) Significance

It was revealed that administration of high-concentration oxygen suppressed superoxide generation in the pathological conditions of ischemia-reperfusion. It was indicated that administration of high-concentration oxygen reduces oxidation stress, and importance of oxygen administration in the whole body management in severe patients was confirmed.

### < Example 8 >

Effect of administration of allopurinol on superoxide generation in a forebrain ischemia reperfusion model

### 1) Experimental conditions

Fourteen male Wister rats (250-300 g) were randomly assigned to the sham group and the allopurinol group. Under isoflurane anesthetization and mechanical ventilation, the superoxide sensor was inserted into the internal jugular vein, and superoxide values were measured continuously. Forebrain ischemia was made by bilateral common carotid artery occlusion-hemorrhagic hypotension. After ischemia was maintained for 10 min, reperfusion was conducted, and the brain tissue and serum were sampled at 120 min after reperfusion. In the allopurinol group, 200 µg/g of allopurinol was intraperitoneally administered 24 hours and 1 hour before creating ischemia, and the equal amount of saline was administered in the sham group.

### 2) Result

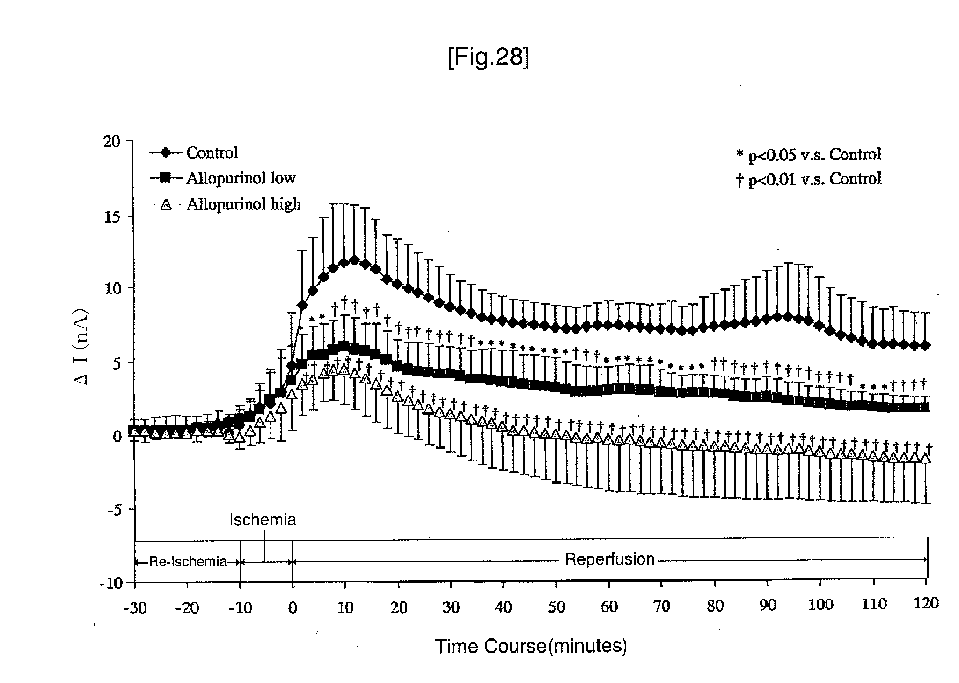

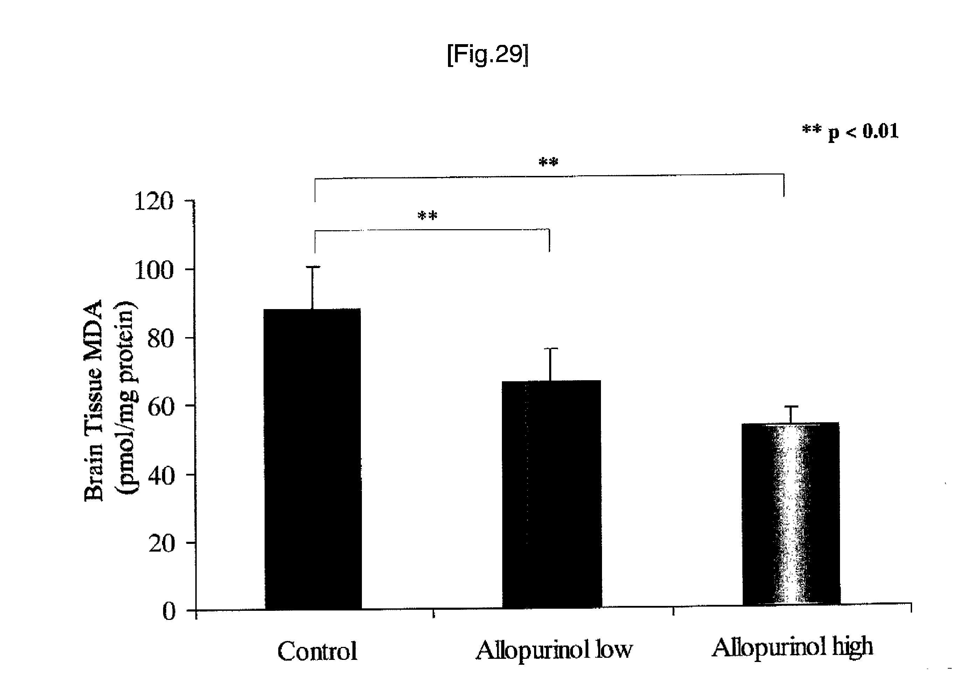

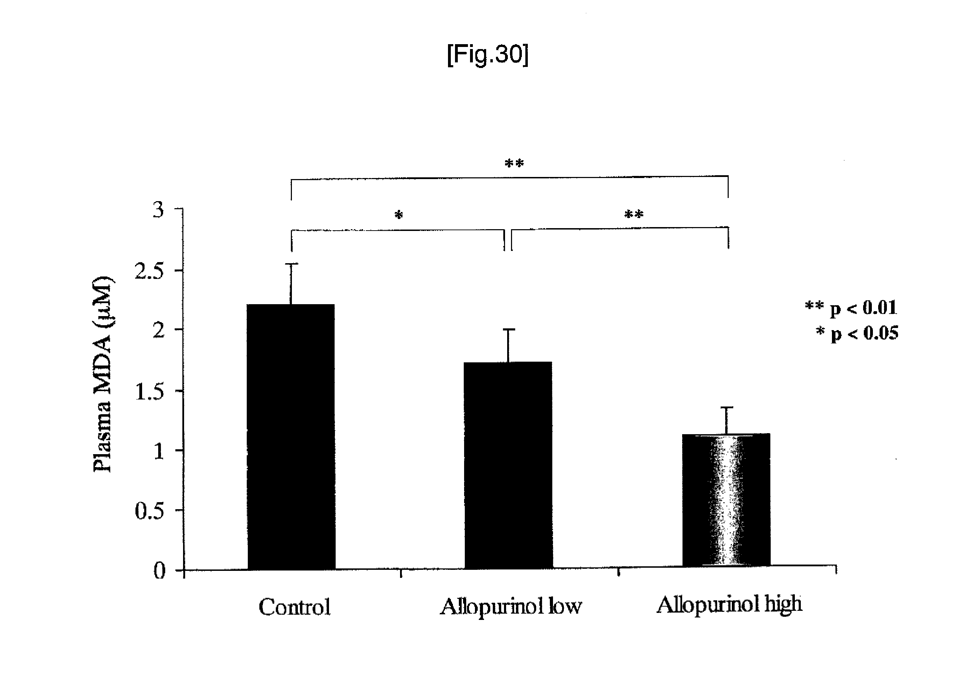

In the allopurinol group, superoxide values during the course were significantly suppressed compared to those in the control group (Fig. 28). MDAs in the brain tissue and in serum were also significantly suppressed in the allopurinol group (Figs. 29, 30).

### 3) Significance

It was revealed that administration of allopurinol, which is a xanthine oxidase inhibitor, suppresses superoxide generation in the pathological conditions of ischemia-reperfusion. It was indicated that administration of allopurinol reduces oxidation stress, and it was confirmed xanthine oxidase is a major source of generation of superoxide at the time of cerebral ischemia reperfusion.

### < Example 9 >

Effect of ulinastatin on superoxide generation in an endotoxinemia model

### 1) Experimental conditions

Fourteen male Wister rats (250-300 g) were randomly assigned to the control group and the ulinastatin group. Under isoflurane anesthetization and mechanical ventilation, the superoxide sensor was inserted into the right atrium, and superoxide values were measured continuously. Endotoxinemia was made by intravenous administration of endotoxin (LPS, 3 µg/g). 5000 units/kg of ulinastatin was intravenously administered just after LPS administration in the ulinastatin group, while the equal amount of saline was administered in the sham group. Blood pressure was recorded until at 6 hours after LPS administration, and blood gas analysis and serum collection were performed every one hour.

### 2) Result

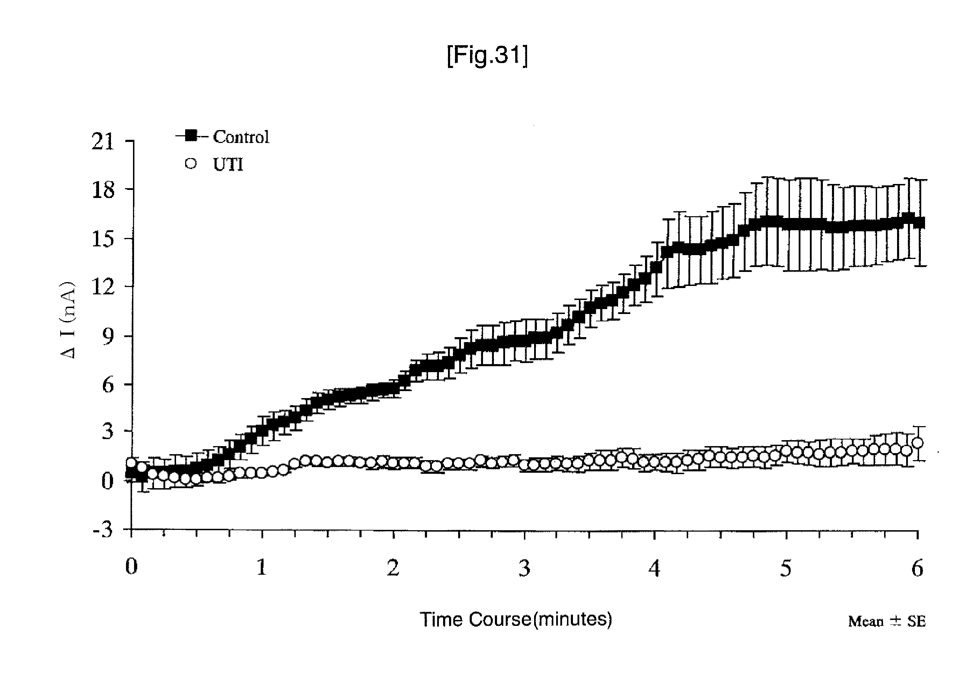

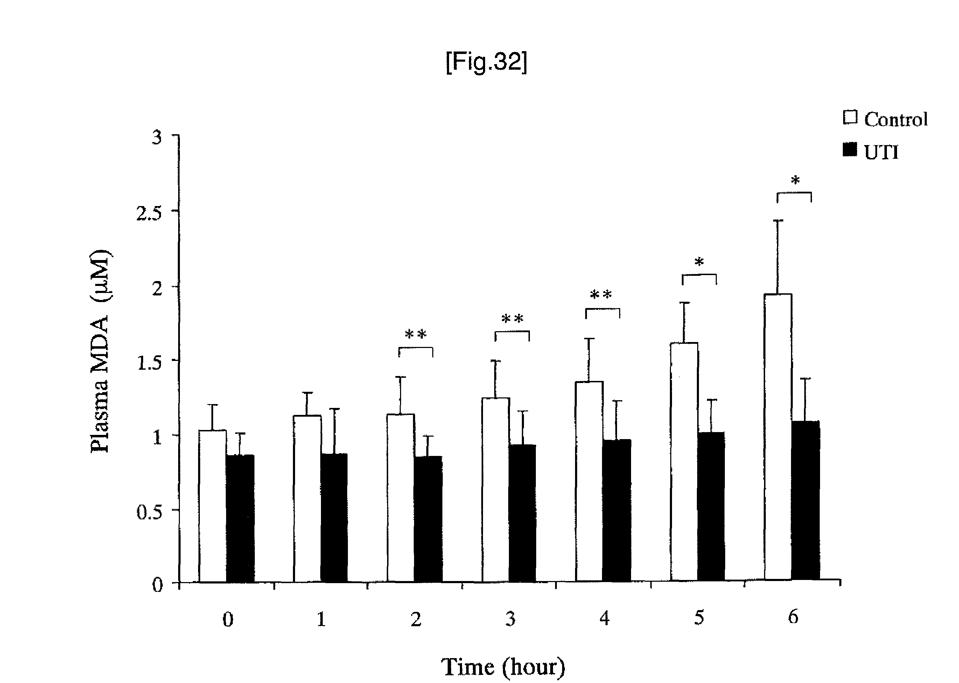

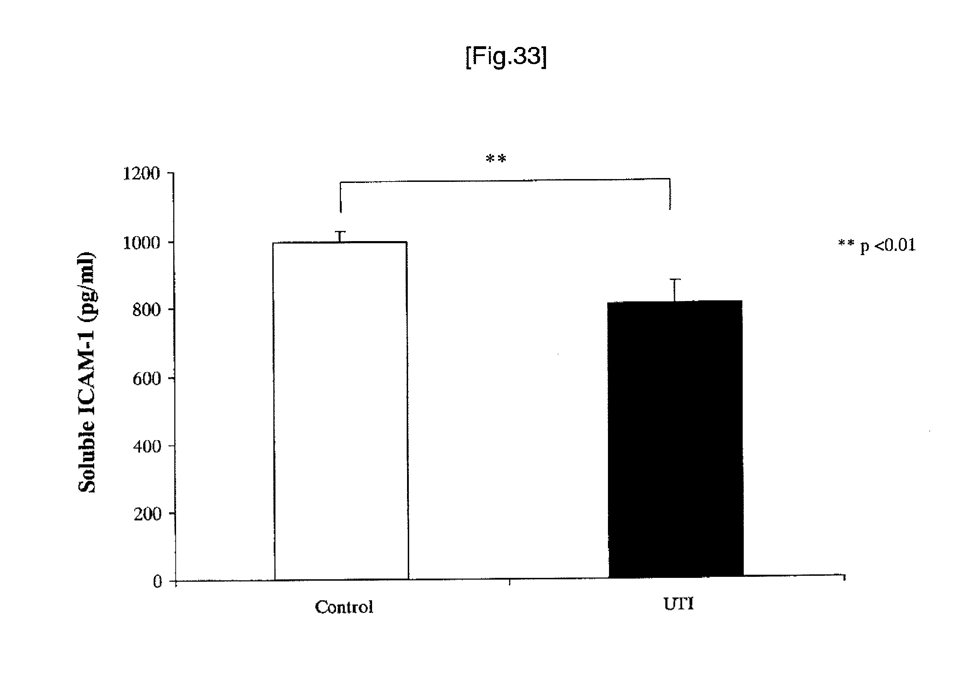

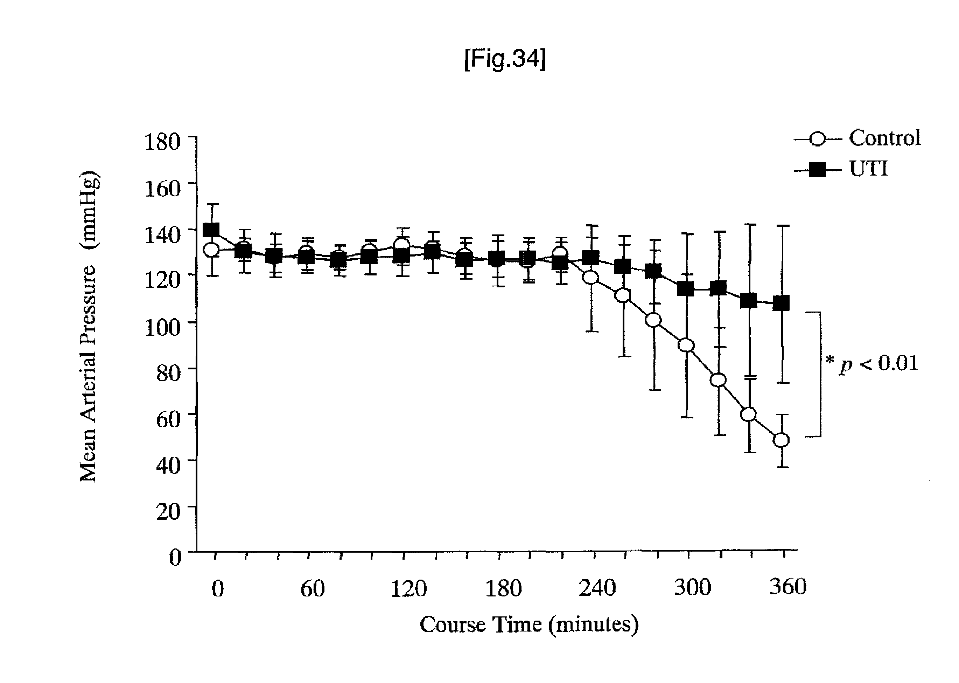

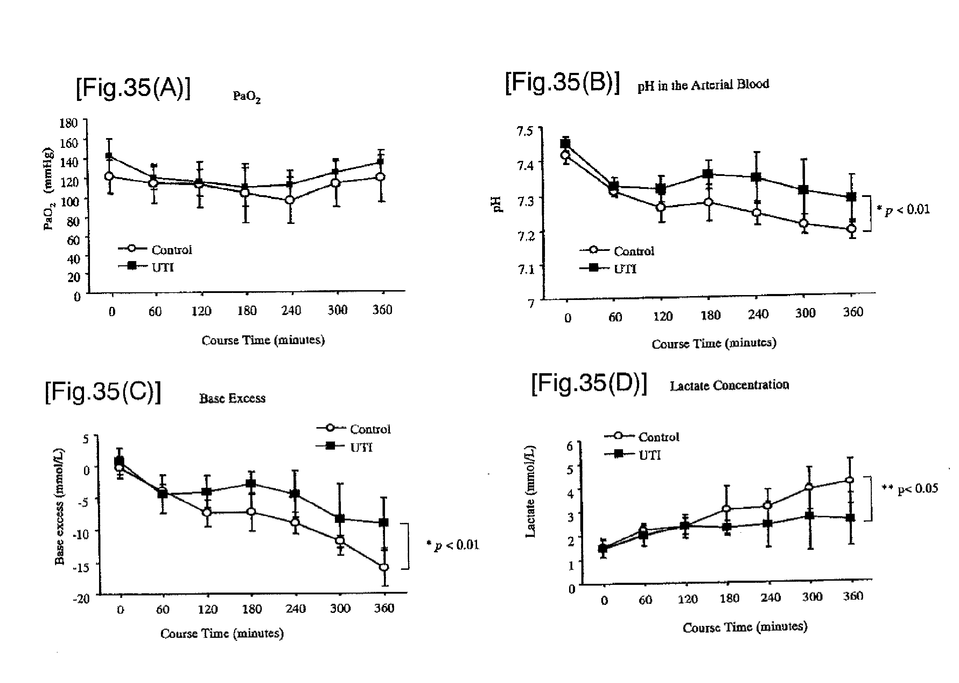

The elevation of superoxide values were observed at 1 hour after LPS administration in the control group, while they were significantly suppressed in the ulinastatin group (Fig. 31). Malondialdehyde (MDA), a lipid peroxidative substance in serum, was also significantly suppressed in the ulinastatin group (Fig. 32). The elevation of soluble ICAM-1, an index of vascular endothelial injury, was observed in the control group, and it was significantly suppressed in the ulinastatin group (Fig. 33). Furthermore, blood pressure (Fig. 34) and lactate acidosis (Fig. 35) were suppressed in the ulinastatin group compared to those in the control group.

### 3) Significance

A protease inhibitor ulinastatin suppressed superoxide generation and lipid peroxidative substance MDA. As a result of this, vascular endothelial injury and shock, and peripheral circulatory failure also improved. Ulinastatin can control oxidation stress, blood vessel injury and circulatory failure in endotoxinemia, and thus can be a treatment drug for endotoxinemia.

### INDUSTRIAL APPLICABILITY

The device of the present invention as described above can diagnose tissue injuries caused by various diseases with a radical sensor provided at the catheter tip end portion, and administer necessary drugs while monitoring the degree of the injuries. Therefore, the device is capable extremely accurate diagnosis and treatment.

Therefore, the device for diagnosing tissue injury of the present invention is extremely useful in diagnosing whether the in vivo tissue conditions are good or not.

## Claims

1. A device for diagnosing tissue injury comprising a catheter insertable into the body, and a radical sensor provided in the catheter, wherein
said radical sensor has a sensor electrode capable of measuring superoxide anion radicals provided at a tip end of said catheter, a lead wire connector for a sensor provided at a basal portion of said catheter, and a lead wire for a sensor for connecting said sensor electrode portion to the lead wire connector for a sensor.

2. The device for diagnosing tissue injury according to Claim 1, further comprising comparative means for comparing a concentration of superoxide anion radicals in blood measured by said sensor electrode with a predetermined threshold to distinguish a tissue injury-based value and a healthy man-based value.

3. The device for diagnosing tissue injury according to Claim 1 or 2, wherein the sensor electrode capable of measuring superoxide anion radicals uses metal porphyrin complex polymerized coating.

4. The device for diagnosing tissue injury according to any one of Claims 1 to 3, wherein transfusion supply means is provided in said catheter, and
said transfusion supply means has tip end members provided at a tip end of said catheter and having a hole capable of discharging transfusion, transfusion lines each of which is connected to each of these tip end members, and transfusion connectors each of which is coupled to each of these transfusion lines and provided at a basal portion of said catheter.

5. The device for diagnosing tissue injury according to Claim 4, wherein at least one of said transfusion lines is coupled to a pressure transducer for venous pressure measurement.

6. The device for diagnosing tissue injury according to Claim 4 or 5, wherein a hole capable of discharging transfusion is also provided at a middle portion of the catheter.

7. The device for diagnosing tissue injury according to any one of Claims 1 to 6, wherein the tissue injury is cerebral ischemia reperfusion injury.

8. The device for diagnosing tissue injury according to any one of Claims 1 to 6, wherein the tissue injury is cerebral ischemia reperfusion injury, and further is cerebral ischemia reperfusion injury exacerbated by hyperglycemia (or diabetes or other disease caused by hyperglycemia).

9. The device for diagnosing tissue injury according to any one of Claims 1 to 6, wherein the tissue injury is cerebral ischemia reperfusion injury and its treatment is drug therapy or low-temperature therapy.

10. The device for diagnosing tissue injury according to any one of Claims 1 to 6, wherein the tissue injury is cerebral ischemia reperfusion injury and its treatment is high-concentration oxygen therapy.

11. The device for diagnosing tissue injury according to any one of Claims 1 to 6, wherein the tissue injury is systemic organ or tissue injury caused by severe infection or sepsis.
